# FASCICULE DE BREVET EUROPEEN

(11) **EP 0 618 209 B1**
(45) Date de publication et mention de la délivrance du brevet: **23.12.1998**
(21) Numéro de dépôt: 94400627.9
(22) Date de dépôt: 24.03.1994
(51) Int. Cl.: C07D 513/04, A61K 31/54

(54) **Pyridothiadiazines, leurs procédés de préparation et les compositions pharmaceutiques qui les contiennent**
Pyridothiadiazine, Verfahren zu ihrer Herstellung und sie enthaltende pharmazeutische Zusammensetzungen
Pyridothiadiazines, processes for their preparation and pharmaceutical compositions containing them

(30) Priorité: 26.03.1993 FR 9303458
(43) Date de publication de la demande: 05.10.1994
(73) Titulaire: ADIR ET COMPAGNIE, 92415 Courbevoie Cédex (FR)
(72) Inventeur: Pirotte, Bernard, B-4680 Oupeye (BE); De Tullio, Pascal, B-4020 Jupille (BE); Masereel, Bernard, B-6890 Libin (BE); Delarge, Jacques, B-4140 Dolembreux (BE); Lepagnol, Jean, F-28210 Chaudon (FR); Renard, Pierre, F-78000 Versailles (FR)

(56) Documents cités:
- GB-A- 1 368 948
- US-A- 3 157 647
- CHEMICAL ABSTRACTS, vol. 91, no. 5, 30 Juillet 1979, Columbus, Ohio, US; abstract no. 39441y, S. K. KOTOVSKAYA ET AL 'Synthesis and properties of pyrido(2,3-e)-1,2,4-thiadiazine 1,1-dioxides' page 631 ; & KHIM.-FARM. ZH. vol. 13, no. 4 , 1979 pages 54 - 57
- CHEMICAL ABSTRACTS, vol. 87, no. 9, 29 Août 1977, Columbus, Ohio, US; abrégé no. 68437Z, K. NODA ET AL 'Pyridothiadiazines' page 562; & JP-A-52 042 897 (HISAMITSU PHARMACEUTICAL)
- CHEMICAL ABSTRACTS, vol. 83, no. 7, 18 Août 1975, Columbus, Ohio, US; abrégé no. 58608J, J. DELARGE ET AL 'Synthesis and pharmacological properties of some N-acylsulfonamides' page 483; & ANN. PHARM. FR. vol. 32, no. 12 , 1974 pages 657 - 667
- Mol.Orbital Stud.Chem.Pharmacol., Sym.(1970);Meeting Date 1969, 262-87. Editor: Lemont B. Kier, Publisher: Springer, New York, N.Y.
- Afinidad(1971), 28(292), 1289-1296

## Description

La présente invention concerne de nouvelles pyridothiadiazines, leurs procédés de préparation et les compositions pharmaceutiques qui les contiennent.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Récemment, les études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545)ou lors du vieillissement cérébral (Brain Research, 1992, 589, 320-326).

Par ailleurs, d'innombrables travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazolepropionic acid") apparait être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs (Brain Research, 1992, 573, 228-234, Behavioral and Neural Biology, 1992, 58, 222-231). De même, les agents nootropes tels que l'aniracetam ont été décrits très récemment comme modulant positivement les récepteurs AMPA des cellules neuronales (Journal of Neurochemistry, 1992, 58, 1199-1204).

La demanderesse a présentement découvert de nouvelles pyridothiadiazines qui possédent de façon surprenante une puissante activité facilitatrice sur le courant AMPA, qui apparaissent donc utiles au clinicien dans le traitement des pathologies impliquant le récepteur à l'AMPA. Pour exemple, il a été montré que la sensibilité du récepteur AMPA diminue lors du vieillissement cérébral (Hippocampus, 1992, 2, 457-468).

On connait de l'art antérieur des pyridothiadiazines décrites dans la publication Kotovskaya et al. Khim. - Farm. 2h. (1979), vol. 13, n° 4, pp 54-57.

D'autres pyridothiadiazines sont également décrites dans la demande de brevet FR 2267 775 comme diurétiques.

Plus particulièrement, l'invention concerne les composés de formule (I) 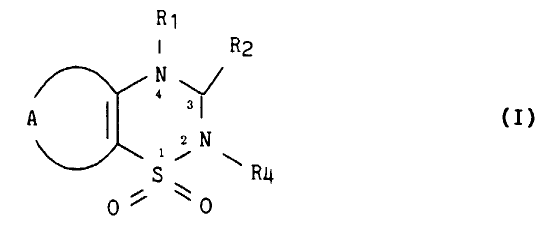 dans laquelle :
- R₁ représente un groupement choisi parmi :
   . hydrogène,
   . R₃ avec R₃ représentant un radical choisi parmi alkyle inférieur, alcényle inférieur et alcyryle inférieur R₃, étant non substitué ou substitué,
   . cycloalkyle,
   . cycloalkyle substitué,
   . cycloalkylalkyle inférieur,
   . cycloalkylalkyle inférieur substitué,
   . acyle inférieur,
   . alkoxy carbonyl inférieur,
   . arylcarbonyl non substitué ou substitué sur le noyau aryle,
   . aryloxycarbonyl non substitué ou substitué sur le noyau aryle,
   ou R₁ forme entre les atomes 3 et 4 du cycle thiadiazinique, présent dans la formule (I), une double liaison ;
- R₂ représente un groupement choisi parmi :
   . hydrogène,
   . R₁₀ avec R₁₀ ayant la même définition que R₃ ci-dessus, R₁₀ étant non substitué ou substitué,
   . R₁₁ avec R₁₁ représentant un groupement choisi parmi cycloalkyle, cycloalkylalkyle inférieur, bicycloalkyle, et bicycloalkylalkyle inférieur, R₁₁ étant non substitué ou substitué,
   . -O-R₁₀ avec R₁₀ tel que défini précédemment,
   . thioxo,
   . S-R₁₂, dans lequel R₁₂ représente un alkyle inférieur, R₁₂ étant non substitué ou substitué,
   . aryle,
   . aryle substitué,
   . et 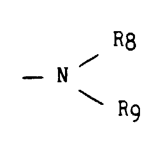 dans lequel R₈ et R₉ représentent, indépendamment l'un de l'autre, un radical choisi parmi :
      . hydrogène,
      . alkyle inférieur,
      . cycloalkyle,
      . cycloalkylalkyle inférieur,
      . un hétérocycle R₁₃ choisi parmi pyrrolidine, pipéridine, pipérazine, morpholine, et thiomorpholine ;
      . ou forment ensemble avec l'atome d'azote qui les porte un hétérocycle R₁₃ tel que défini précédemment,
- R₄ représente un groupement choisi parmi :
   . hydrogène,
   . R₁₄, R₁₄ ayant la même définition que R₃ ci-dessus, R₁₄ étant non substitué ou substitué,
   . cycloalkyle,
   . aryle,
   . aryle substitué,
   . acyle inférieur,
   . alkoxycarbonyl inférieur,
   . arylcarbonyl non substitué ou substitué sur le noyau aryle,
   . aryloxycarbonyl non substitué ou substitué sur le noyau aryle,
   ou
   R₄ forme entre les atomes 2 et 3 du cycle thiadiazinique, présent dans la formule (I), une double liaison ;
- A forme avec les 2 atomes de carbone qui le portent un noyau pyridinique choisi parmi les groupements A₁, A₂, A₃ et A₄ : 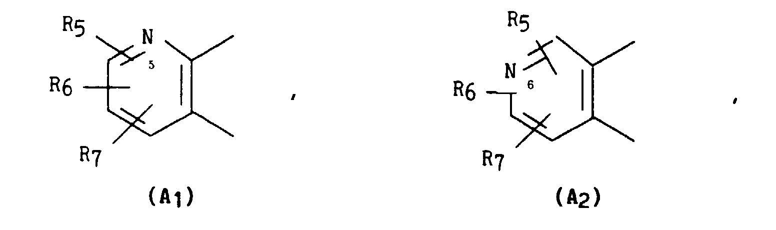 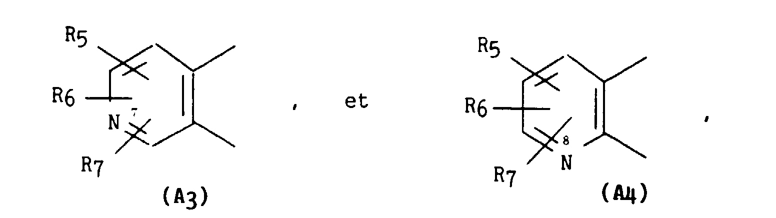 dans lesquels R₅, R₆ et R₇ représentent, indépendamment l'un de l'autre un radical choisi parmi : hydrogène, halogène, alkyle inférieur, hydroxy, thiol, alkoxy inférieur, alkyle-thio inférieur, trifluorométhyle, carboxyle, acyle inférieur, aryle, arylalkyle inférieur, amino, alkylamino inférieur et dialkylamino inférieur,
   étant entendu que lorsque A représente un groupement de formule A₁ non substitué, R₂ représente un atome d'hydrogène, un radical amino ou un radical méthyle, et R₅, R₆, R₇ représentent simultanément des atomes d'hydrogène, alors R₁ ne peut pas être un atome d'hydrogène ou un radical méthyle,
   étant entendu que le composé de formule (I) ne peut représenter le 4H-pyrido[4,3-e]-1,2,4-thiadiazine-1,1-dioxyde,
   étant entendu que, sauf précisions contraires,
   . "alkyle inférieur", "alkoxy inférieur", "acyle inférieur" et "alkyl thio inférieur" signifient des groupements linéaires ou ramifiés de 1 à 6 atomes de carbone,
   . "alcényl inférieur" signifie un groupement linéaire ou ramifié de 2 à 6 atomes de carbone comprenant de 1 à 2 double liaisons,
   . "alcynyl inférieur" signifie un groupement linéaire ou ramifié de 2 à 6 atomes de carbone comprenant de 1 à 2 triple liaisons,
   . "cycloalkyle" signifie un groupement cyclique de 3 à 8 atomes de carbone,
   . "bicycloalkyle" signifie un groupement bicyclique de 6 à 12 atomes de carbone,
   . "aryle" signifie phényle ou naphtyle,
   . "substitué" associé aux groupements R₃, R₁₀, et R₁₄, signifie que ces groupements ainsi qualifiés sont substitués par un ou plusieurs radicaux choisis parmi halogène, hydroxy, et alkoxy inférieur,
   . "substitué" associé aux groupements "cycloalkyle", "cycloalkylalkyle", et R₁₁, signifie que ce groupement ainsi qualifié est substitué par un ou plusieurs radicaux ou groupes choisis parmi oxo, hydroxy, alkyle inférieur, et alkoxy inférieur,
   . "substitué" associé au groupement aryle signifie que ce groupement ainsi qualifié est substitué par un ou plusieurs radicaux choisis parmi halogène, hydroxy, alkyle inférieur, alkoxy inférieur, et trifluorométhyle,
- leurs isomères optiques,
- et leurs sels d'addition à un acide ou à une base pharmaceutiquement acceptable.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

Un aspect de l'invention constitue le procédé de préparation des composés de formule (I) caractérisé en ce que l'on fait réagir un composé de formule (II) : 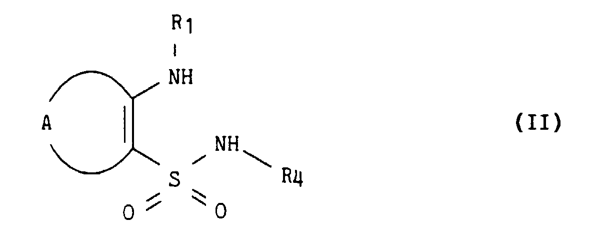 dans laquelle R₁, R₄ et A sont tels que définis dans la formule (I),
a) - soit avec un composé de formule (III) 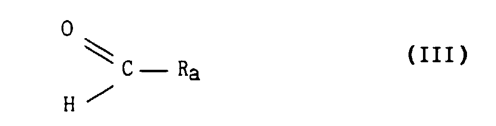 dans laquelle Rₐ représente un groupement R₁₀, R₁₁, aryle, ou aryle substitué, tels que définis dans la formule (I),
   ou, à chaud, avec un composé de formule (IV) : 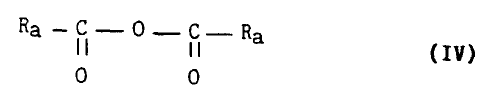 pour obtenir les composés de formule (I/a) : 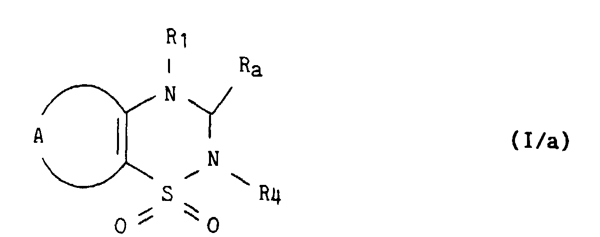 dans laquelle R₁, R₄, Rₐ et A sont tels que définis précédemment,
   cas particulier des composés de formule (I) dans lesquels R₂ représente un groupement Rₐ,
   - soit avec le paraformaldéhyde, l'orthoformiate d'éthyle, ou un anhydride mixte acide formique - acide acétique, pour obtenir les composés de formule (I/b) : 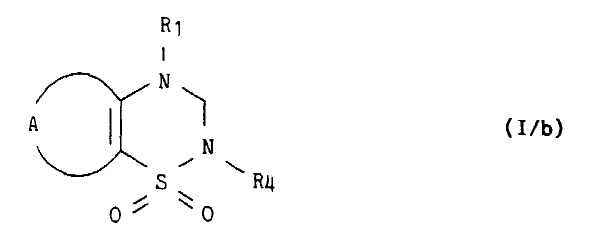 dans laquelle R₁, R₄ et A sont tels que définis précédemment,
   cas particulier des composés de formule (I) dans lesquels R₂ représente un atome d'hydrogène,
   ou bien,
b) - avec de l'urée pour conduire aux composés de formule (V) : 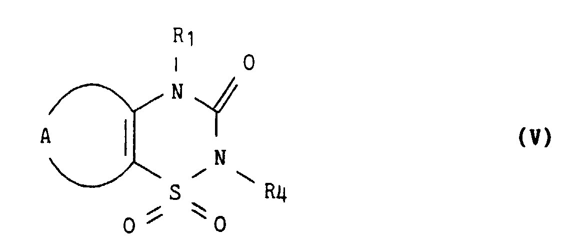 dans laquelle R₁, R₄, et A sont tels que décrits précédemment, qui sont ensuite :
   * salifiés puis mis en réaction avec un composé de formule (VI) :

      R₁₀―Hal (VI)

      dans laquelle R₁₀ est tel que défini dans la formule (I) et Hal représente un atome d'halogène,
      pour obtenir un composé de formule (I/c) : 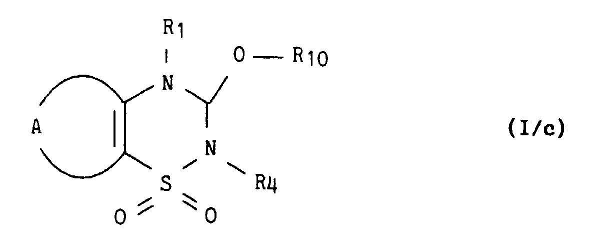 dans laquelle R₁, R₄, R₁₀ et A sont tels que définis précédemment, cas particulier des composés de formule (I) dans lesquels R₂ représente un groupement -O-R₁₀,
   * ou bien soumis à un agent de thionation pour obtenir le composé de formule (I/d) : 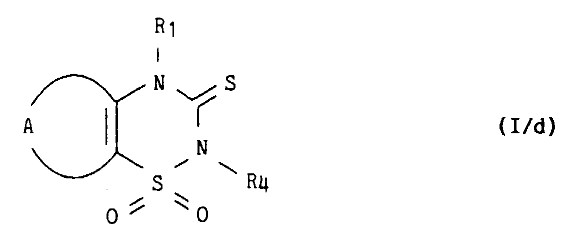 dans laquelle R₁, R₄, et A sont tels que définis précédemment, cas particulier des composés de formule (I) dans lesquels R₂ représente un groupement thioxo, composé de formule (I/d) qui est mis en réaction avec un composé de formule (VI) :

      Hal'―R₁₂ (VI)

      dans laquelle R₁₂ est tel que défini dans la formule (I) et Hal' représente un atome d'halogène pour obtenir le composé de formule (I/e) : 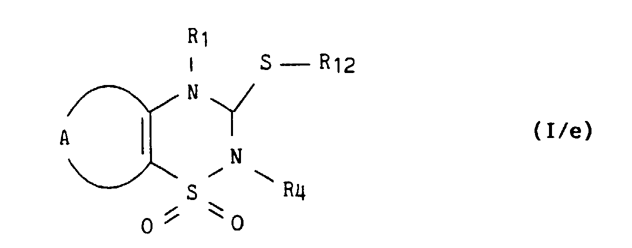 dans laquelle R₁, R₄, R₁₂, et A sont tels que définis précédemment, cas particulier des composés de formule (I) dans lesquels R₂ représente un groupement alkylthio,
   composés de formule (I/e) qui peuvent être ensuite soumis à une amine de formule (VII) : 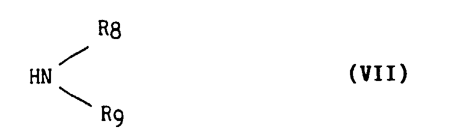 dans laquelle R₈ et R₉ sont tels que définis dans la formule (I) pour obtenir les composés de formule (I/f) : 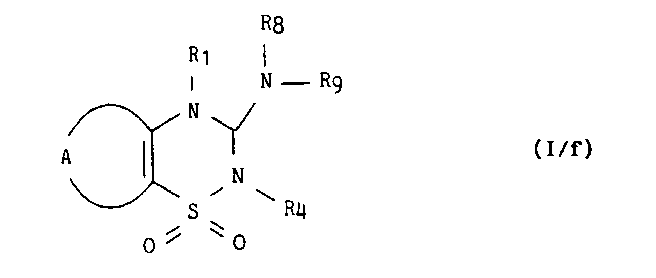 dans laquelle R₁, R₄, R₈, R₉, et A sont tels que définis précédemment, cas particulier des composés de formule (I) dans lesquels R₂ représente un groupement -NR₈R₉, les composés de formule (I/a) à (I/f) formant l'ensemble des composés de formule (I),
   étant entendu que les composés de formule (I/a), (I/b), (I/c), (I/e) et (I/f) peuvent être, le cas échéant, réduits sélectivement afin d'obtenir les composés de formule (I) correspondants où la liaison entre les atomes 2 et 3, ou entre les atomes 3 et 4, du cycle thiadiazinique est saturée.
   les composés de formule (I) pouvant être :
   - purifiés suivant une ou plusieurs méthodes de purification choisies parmi la cristallisation, la chromatographie sur colonne de silice, l'extraction, la filtration, et le passage sur charbon ou résine,
   - séparés, sous forme pure ou sous forme de mélange, en leurs éventuels isomères optiques,
   - et salifiés par un acide ou une base pharmaceutiquement acceptable.

L'invention comprend également le procédé d'obtention des composés de formule (I/a) : 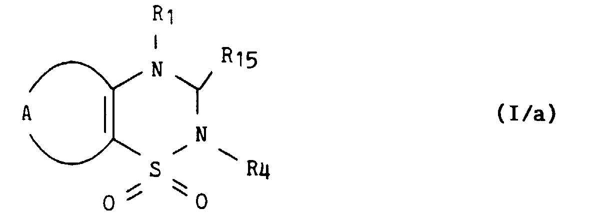 dans laquelle R₁, R₄, et A sont tels que définis dans la formule (I), et R₁₅ représente un groupement R₁₀ ou R₁₁ tels que définis dans la formule (I),
caractérisé en ce que :
on soumet un composé de formule (II/a) : 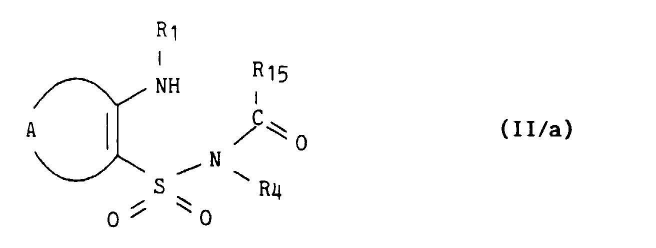 dans laquelle R₁, R₄, R₁₅ et A sont tels que définis précédemment à un agent cyclisant,
afin d'obtenir le composé de formule (I/a) correspondant,
les composés de formule (I/a) ainsi obtenus pouvant être :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et salifiés par un acide ou une base pharmaceutiquement acceptable.

Un autre aspect de l'invention est aussi le procédé de préparation des composés de formule (I/g) : 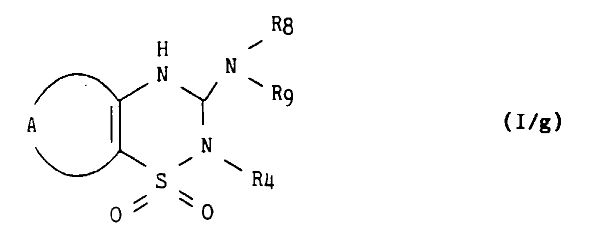 dans laquelle R₄, R₈, R₉, et A sont tels que définis dans la formule (I), cas particulier des composés de formule (I) dans lesquels R₂ représente un groupement -NR₈R₉ et R₁ représente un atome d'hydrogène,
caractérisé en ce que :
- soit on fait réagir à chaud, en milieu basique, un composé de formule (II/b) : 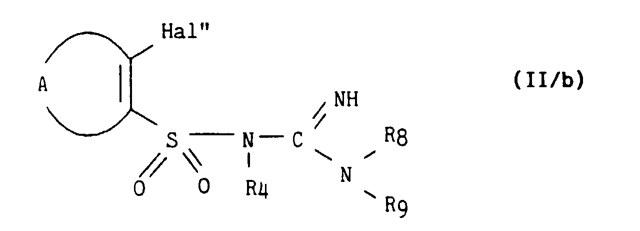 dans laquelle R₄, R₈, R₉, et A sont tels que définis précédemment, et Hal" représente un atome d'halogène,
- soit on fait réagir un composé de formule (II) : 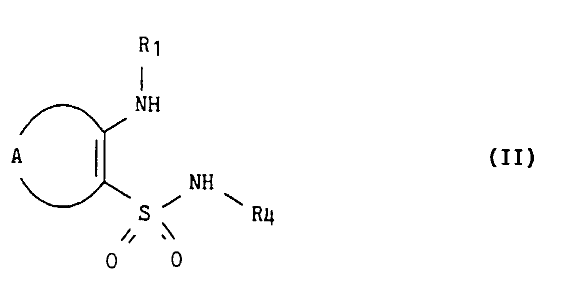 dans laquelle R₁, R₄, et A sont tels que définis précédemment, avec un composé de formule (VIII) : 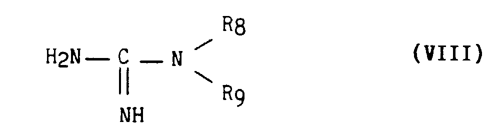 dans laquelle R₈ et R₉ sont tels que définis précédemment,
afin d'obtenir le composé de formule (I/g) correspondant,
les composés de formule (I/g) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et salifiés par un acide ou une base pharmaceutiquement acceptable.

L'invention concerne également le procédé de préparation des composés de formule (I/h) 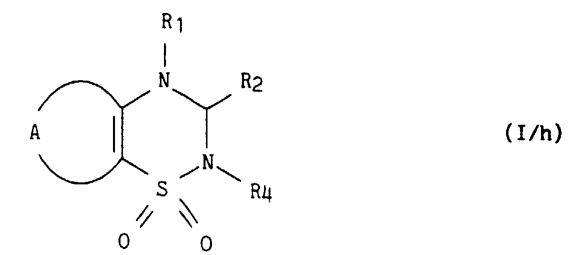 dans laquelle R₁, R₄, et A ont la même signification que dans la formule (I), étant entendu qu'au moins un des substituants R₁ ou R₄ est différent d'un atome d'hydrogène, caractérisé en ce que,
on fait réagir un composé de formula (I/i) : 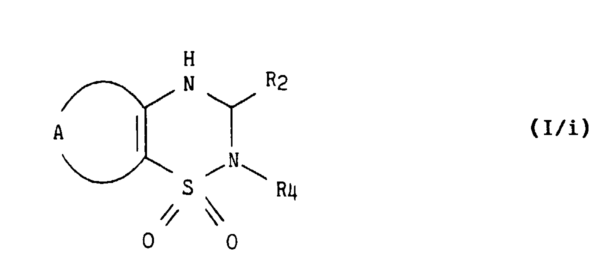 dans laquelle R₂, R₄, et A sont tels que définis précédemment avec un composé de formule (XI) :

R₁'―X (XI)

dans laquelle R₁' a la même signification que R₁ tel que défini dans la formule (I) à l'exception de l'hydrogène et X représente un groupement partant,
et/ou, lorsque R₄ représente un atome d'hydrogène, avec un composé de formule (XII) :

R₄'―X' (XII)

dans laquelle R₄' a la même signification que R₄ tel que défini dans la formule (I) à l'exception de l'hydrogène et X' représente un groupement partant,
les composés de formule (I/h) ainsi obtenus pouvant être :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange en leurs éventuels isomères optiques,
- et salifiés par un acide ou une base pharmaceutiquement acceptable.

Les matières premières utilisées dans les procédés précédents sont soit commerciales, soit aisément accessibles à l'homme du métier selon des procédés connus dans la littérature ou proposés lors des exemples de préparation décrits ci-après.
Les composés de formule (II) sont par exemple aisément accessibles à l'homme du métier par réaction d'un composé de formule (IXa) : 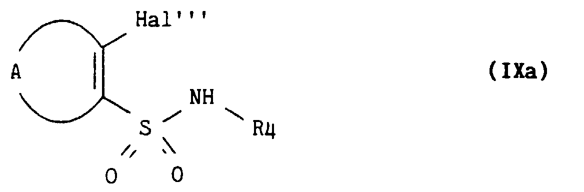 dans laquelle R₄ et A sont tels que définis dans la formule (I) et Hal''' représente un atome d'halogène,
avec une amine de formule (X) :

H₂N―R₁ (X)

dans laquelle R₁ est tel que défini dans la formule (I).

Les composés de formule (II/a) sont par exemple aisément obtenus par l'homme du métier par réaction d'un composé de formule (IXb) : 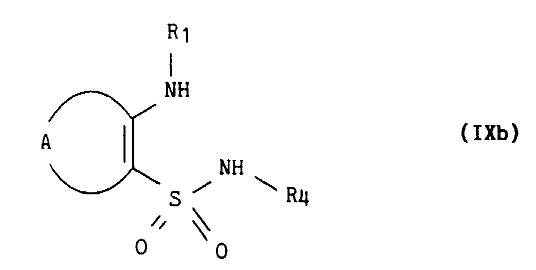 dans laquelle R₁ et A sont tels que décrits dans la formule (I),
avec un composé de formule (XI) : 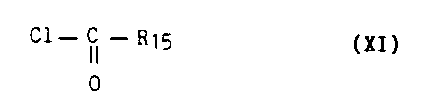 ou de formule (XII) : 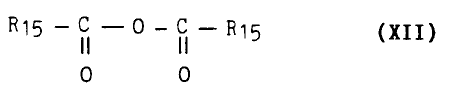 dans lesquelles R₁₅ représente un groupement R₁₀ ou R₁₁ tels que définis dans la formule (I), éventuellement suivie d'une hydrolyse ménagée, lorsque le produit de la réaction a également été acylé sur l'azote portant le groupement R₁.

Les composés de formule (II/b) sont également, par exemple, aisément accessibles à l'homme du métier par réaction d'un composé de formule (IXc) : 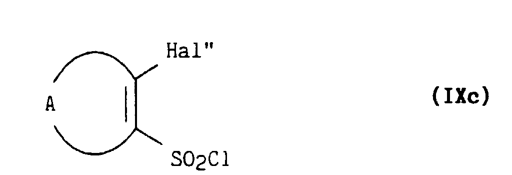 dans laquelle A est tel que défini précédemment et Hal" représente un atome d'halogène,
avec un composé de formule (XIV) : 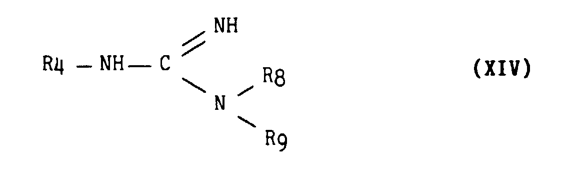 dans laquelle R₄, R₈ et R₉ sont tels que définis dans la formule (I).

Les pyrido[3,2-e][1,2,4]thiadiazine 1,1-dioxydes sont également accessibles à partir de 2-chloro-3-nitro-pyridines diversement substituées.

D'une façon générale, l'introduction d'un groupement nitro (en position 3, en position 5, en positions 3 et 5) s'effectue au départ d'une 2-aminopyridine. Lorsque la position 5 est occupée, seul le dérivé 3-nitro est obtenu. Lorsque la position 5 est libre, les composés mononitro en 3 ou 5 peuvent être obtenus et séparés, ainsi que le composé dinitro en 3 et 5.

Lorsque la 2-aminopyridine n'est pas disponible commercialement, une 2-hydroxypyridine peut dans certains cas être nitrée de façon identique. D'autre part, les 2-chloropyridines donnent accès éventuellement aux 2-aminopyridines.

Les substituants du noyau pyrido aisément accessibles sont les radicaux alkyles en particulier méthyle, les radicaux amino, alkylamino, et dialkylamino par réduction d'une fonction nitro, les radicaux trifluorométhyles, les radicaux halogénés, en particulier le chlore et le brome, les radicaux alkoxy, en particulier méthoxy, les radicaux carboxy et acyles.

Les composés de la présente invention présentent des propriétés pharmacologiques intéressantes puisqu'ils peuvent potentialiser sélectivement les phénomènes électrophysiologiques excitateurs induits par l'AMPA, soit dans l'ovocyte de Xenopus exprimant les récepteurs au glutamate par injection d'ARNm de cortex de Rat, soit dans l'hippocampe lors de stimulation électrique des voies de neurotransmission glutamatergique.

Comme le montrent les études électrophysiologiques conduites vis-à-vis de l'excitabilité induite par l'AMPA exprimé dans l'ovocyte de Xenopus ou présent naturellement dans l'hippocampe. Ces effets sont toujours supérieurs à ceux des composés pris en référence et confèrent aux composés de l'invention des potentialités pharmacologiques et thérapeutiques vis-à-vis des troubles induits par le dysfonctionnement de la neurotransmission glutamatergique et notamment liés au récepteur AMPA.

La neurotransmission glutamatergique est désormais largement démontrée comme cruciale pour les processus physiologiques de l'apprentissage et de la mémoire, et plus largement pour les processus gouvernant les facultés d'attention de concentration et de vigilance. Plus particulièrement, le sous-type réceptoriel dénommé AMPA semble jouer un rôle fondamental pour ces processus. Les composés de la présente invention ont montré des effets pharmacologiques intéressants puisqu'ils peuvent sélectivement faciliter l'activation de ce récepteur AMPA. Ces effets sont, de façon surprenante, beaucoup plus intenses que ceux des composés de référence : le Diazoxide et l'Aniracetam.

Les composés de l'invention sont donc utiles en tant qu'agents facilitateurs des récepteurs AMPA et constituent donc des agents thérapeutiques pour le traitement et la prévention des pathologies liées au dysfonctionnement de la neurotransmission glutamatergique telles que :
- les désordres mnémocognitifs associés à l'âge et aux syndromes anxieux ou dépressifs,
- les maladies neurodégénératives progressives comme par exemple la maladie d'Alzheimer, la maladie de Pick, la chorée d'Huntington et la schizophrénie,
- les séquelles des maladies neurodégénératives aigües comme par exemple l'ischémie et l'épilepsie.

Les composés se sont révélés également intéressants pour le traitement des pathologies de type diabétique, liées au dysfonctionnement de l'insulino sécrétion qui a été décrite récemment comme régulée par les récepteurs AMPA (British Journal of Pharmacology, 1992, 106, 354-359).

La présente invention a également pour objet les compositions pharmaceutiques contenant un composé de formule générale (I), ou un de ses sels à un acide ou à une base pharmaceutiquement acceptable, en combinaison avec un ou plusieurs excipients ou véhicules pharmaceutiquement acceptables.

Parmi les compositions pharmaceutiques selon l'invention, on pourra citer, à titre d'exemples et de façon non limitative, celles qui conviennent à une administration par voie orale, rectale, nasale ou parentérale et notamment les comprimés, les dragées, les gélules, les paquets, les sachets, les granules, les pilules, les granulés, les suppositoires, les crèmes, les pommades, les aérosols, les capsules, les gels dermiques et les solutions injectables ou buvables.

La posologie varie d'un individu à l'autre, selon l'âge, le poids, et le sexe du malade, la voie d'administration choisie, la nature et l'intensité de l'affection. Les doses utilisées s'échelonnent entre 1 et 500 mg pour un traitement, divisibles en 1 à 3 prises par 24 h.

Les préparations suivantes sont utiles dans la préparation des composés de l'invention. Elles ne font pas partie de l'invention.
Abréviations utilisées : P.F. (point de fusion), Rdt (Rendement), eq. (équivalent).

### PREPARATION 1 :

### (3-AMINOPYRID-2-YL)SULFONAMIDE

Le composé du titre est obtenu selon la technique décrite dans la littérature [R. Lejeune et al., J. Pharm. Bel. (1984), vol. 39 pp. 217-224]

### PREPARATION 2 :

### (4-AMINOPYRID-3-YL)SULFONAMIDE

15 g de (4-chloropyrid-3-yl)sulfonamide sont mis en solution dans 150 cm³ d'ammoniaque concentré. La solution limpide est saturée par un courant gazeux d'ammoniaque pendant une demi heure juste avant d'être introduite dans une bombe scellée. La bombe est placée à 150°C pendant 18 heures.
Ensuite, le milieu réactionnel est refroidi et concentré au rotavapor jusqu'à un volume de 30 cm³. Le précipité obtenu est recueilli sur filtre, lavé avec un peu d'eau et séché.
Rdt : 75-80 %
P.F. : 212-215°C

### PREPARATION 3 :

### (4-ISOPROPYLAMINOPYRID-3-YL)SULFONAMIDE

10 g de (4-chloropyrid-3-yl)sulfonamide sont dissous dans 50 cm³ de méthanol et 50 cm³ d'isopropylamine. La solution est introduite dans une bombe scellée et portée à 120°C pendant 18 heures. Après refroidissement, le milieu réactionnel est concentré à siccité sous vide (rotavapor) et le résidu est repris par 200 cm³ d'eau. Le précipité correspondant du composé du titre est recueilli sur filtre et lavé à l'eau. Le précipité est redissous dans 100 cm³ de NaOH dilué. La solution est décolorée au charbon, filtrée, puis acidifée à pH 6,5-7. Le précipité cristallin obtenu est recueilli sur filtre, lavé à l'eau et séché.
Rdt : 45-50 %
P.F. : 168-171°C.

### PREPARATIONS 4 ET 5 :

Ces préparations sont obtenues selon la technique décrite Eur. J. med. Chem. (1980), vol. 15 N°4, pp 299-304.

### PREPARATION 4 :

### (4-CYCLOHEXYLAMINOPYRID-3-YL)SULFONAMIDE

### PREPARATION 5 :

### (4-CYCLOOCTYLAMINOPYRID-3-YL)SULFONAMIDE

### PREPARATION 6 :

### (2-AMINOPYRID-3-YL)SULFONAMIDE

### Stade A : (2-chloropyrid-3-yl)sulfonamide

20 g de 3-amino-2-chloropyridine (commercial) sont dissous dans 200 cm³ d'acide acétique glacial et 74 cm³ d'HCl concentré. Cette solution est refroidie dans un bain de glace et de sel à -10°C et diazotée par addition progressive de 20 cm³ d'une solution aqueuse contenant 13 g de nitrite sodique.
D'autre part, à une solution d'acide acétique (320 cm³) saturée en anhydride sulfureux, on ajoute 8 g de CuCl₂ dissous dans 40 cm³ d'eau, puis la solution de sel de diazonium formé précédemment. Après quelques minutes, la suspension est concentrée à siccité et le résidu est repris par un mélange eau-glace, puis épuisée au diéthyléther.
La phase éthérée est séchée sur MgSO₄ anhydre, filtrée, puis concentrée sous vide. Le résidu de chlorure de (2-chloropyrid-3-yl)sulfonyle est dissous dans 30 cm³ d'acétone et additionné progressivement à 30 cm³ d'ammoniaque concentré. Après quelques minutes. sous agitation, le mélange est concentré jusqu'à siccité au rotavapor et le résidu est repris par 50 cm³ d'eau. Le précipité est recueilli sur filtre, lavé avec de l'eau et séché.
Rdt : 60-65 %
P.F. : 185-187°C.

### Stade B : (2-aminopyrid-3-yl)sulfonamide

En procédant comme dans la préparation 2 mais en remplaçant le (4-chloropyrid-3-yl)sulfonamide par le composé obtenu au stade précédent, on obtient le (2-aminopyrid-3-yl)sulfonamide.
Rdt : 75-80 %
P.F. : 170-172°C

### PREPARATION 7 :

### N-ISOPROPYL-(4-AMINOPYRID-3-YL)SULFONAMIDE

### Stade A : N-isopropyl-(4-chloropyrid-3-yl)sulfonamide

7,5 g d'acide (4-hydroxypyrid-3-yl)sulfonique, 30 g de pentachlorure de phosphore et 5 cm³ d'oxychlorure de phosphore sont portés à reflux pendant 5 heures. Après refroidissement et concentration sous vide (rotavapor), le résidu huileux est versé sur glace et la phase aqueuse est extraite à 3 reprises par du diéthyléther. La phase éthérée est séchée sur MgSO₄ anhydre, filtrée, puis concentrée sous vide, le résidu de chlorure de (4-chloropyrid-3-yl)sulfonyle est dissous dans 20 cm³ de dioxane et versé sur une solution d'isopropylamine (2,6 cm³) et de triéthylamine (5 cm³) dans le dioxane (40 cm³). Après 1/2 heure à température ambiante, la suspension obtenue est concentrée sous vide (rotavapor) à siccité. Le résidu est repris par 100 cm³ d'eau. Après 1/2 heure d'agitation, le précipité cristallin obtenu est recueilli sur filtre, lavé à l'eau et séché.
Rdt : 50-55 %
P.F. : 109-111°C.

### Stade B : N-isopropyl-(4-aminopyrid-3-yl)sulfonamide

En procédant comme dans la préparation 2 mais en partant du composé obtenu au stade précédent, on obtient le N-isopropyl-(4-aminopyrid-3-yl)sulfonamide
Rdt : 70-75%
P.F : 190-193°C

### PREPARATION 8 :

### (2-METHYLAMINOPYRID-3-YL)SULFONAMIDE

10 g de (2-chloropyrid-3-yl)sulfonamide (obtenu au stade A de la préparation 6) sont dissous dans 100 cm³ de solution aqueuse de méthylamine à 40 % et la solution est introduite dans une bombe scellée. La bombe est placée à 150°C pendant 18 heures. Après refroidissement, le milieu réactionnel est concentré sous vide (rotavapor) à un volume de 30 cm³. Le précipité cristallin correspondant au composé du titre est recueilli sur filtre, lavé à l'eau et séché.
Rdt : 80-85 %
P.F. : 167-169°C

### PREPARATION 9

**N-PENTYL-(4-AMINOPYRID-3-YL)SULFONAMIDE**

### Stade A : N-pentyl-(4-chloropyrid-3-yl)sulfonamide

En procédant comme dans le stade A de la préparation 7, mais en remplaçant l'isopropylamine par 3,5 cm³ de pentylamine, on obtient le composé du titre (huile).
Rdt : 50 %

### Stade B : N-pentyl-(4-aminopyrid-3-yl)sulfonamide

En procédant comme dans la préparation 2 mais en partant du composé obtenu au stade précédent, on obtient le composé du titre qui est purifié par dissolution dans HCl 0,1N, filtration de l'insoluble éventuel, décoloration au charbon absorbant et neutralisation du filtrat par NaOH dilué jusqu'à pH 7,5. Le précipité obtenu est recueilli sur filtre, lavé à l'eau et séché.
Rdt : 30-35 %
P.F. : 139-142°C

### PREPARATION 10 :

### N-CYCLOHEXYL-(4-AMINOPYRID-3-YL)SULFONAMIDE

En procédant comme dans la préparation 7, mais en remplaçant l'isopropylamine par 3,4 cm³ de cyclohexylamine, on obtient le composé du titre.

### Stade A : N-cyclohexyl-(4-chloropyrid-3-yl)sulfonamide

Rdt : 50 %
P.F. : 123-125°C

### Stade B : N-cyclohexyl-(4-aminopyrid-3-yl)sulfonamide

Rdt : 75-80 %
P.F. : 197-200°C

### PREPARATION 11 :

### N-PHENYL-(4-AMINOPYRID-3-YL)SULFONAMIDE

En procédant comme dans la préparation 9 mais en remplaçant la pentylamine par 2,8 cm³ d'aniline, on obtient le composé du titre.

### Stade A : N-phényl-(4-chloropyrid-3-yl)sulfonamide

Rdt : 30-35 %
P.F. : 178-180°C

### Stade B : N-phényl-(4-aminopyrid-3-yl)sulfonamide

Rdt : 60-65 %
P.F. : 231-236°C

### PREPARATION 12 :

### N-METHYL-(4-AMINOPYRID-3-YL)SULFONAMIDE

### Stade A : N-méthyl-(4-chloropyrid-3-yl)sulfonamide

10 g d'acide (4-hydroxypyrid-3-yl)sulfonique, 30 g de pentachlorure de phosphore et 5 cm³ d'oxychlorure de phosphore sont portés à reflux pendant 5 heures.
Après refroidissement, et concentration sous vide (rotavapor), le résidu huileux est versé sur glace et la phase aqueuse est extraite à 3 reprises par du diéthyléther. La phase éthérée est séchée sur MgSO₄ anhydre, filtrée, puis concentrée sous vide, le résidu de chlorure de (4-chloropyrid-3-yl)sulfonyle est dissous dans 20 cm³ de dioxane.
Cette solution est versée lentement sur 100 cm³ de solution aqueuse de méthylamine à 10 %. La solution est concentrée sous vide (rotavapor) jusqu'à un volume de 40 cm³. Le précipité cristallin est recueilli sur filtre, lavé à l'eau et séché.
Rdt : 70-75 %
P.F. : 187-190°C.

### Stade B : N-méthyl-(4-aminopyrid-3-yl)sulfonamide

En procédant comme dans la préparation 2 mais en partant du composé obtenu au stade précédent, on obtient le composé du titre.
Rdt : 80-85 %
P.F. : 168-170°C.

### PREPARATION 13 :

### (4-METHYLAMINOPYRID-3-YL)SULFONAMIDE

10 g de (4-chloropyrid-3-yl)sulfonamide sont dissous dans 100 cm³ de solution aqueuse de méthylamine à 40 % et la solution est introduite dans une bombe scellée. La bombe est placée à 150°C pendant 18 heures. Après refroidissement, le milieu réactionnel est concentré sous vide (rotavapor) à un volume de 30 cm³. Le précipité cristallin correspondant au composé du titre est recueilli sur filtre, lavé à l'eau et séché.
Rdt : 85-90 %
P.F. 251-254°C.

### PREPARATION 14 :

### (4-CHLOROPYRID-3-YL)SULFONYLGUANIDINE

A partir de 5 g d'acide (4-hydroxypyrid-3-yl)sulfonique, on prépare le chlorure de (4-chloropyrid-3-yl)sulfonyle selon la technique décrite dans la préparation 12. Celui-ci est dissous dans 100 cm³ d'éther, puis mis sous forte agitation en présence de 25 cm³ d'une solution de NaOH 2N contenant 4,25 g de carbonate de guanidine (2 éq. de guanidine). Après une heure de réaction, le précipité blanc correspondant au composé du titre est recueilli sur filtre, lavé à l'eau et séché.
Rdt : 15-20 %
P.F. : 174-177°C.

### PREPARATION 15 :

### (2,4-DICHLOROPYRID-5-YL)SULFONYLGUANIDINE

### Stade A : acide (2,4-dihydroxypyrid-5-yl)sulfonique

A température voisine de -10°C, on ajoute lentement 5 g de 2,4-dihydroxypyridine à 12,5 cm³ d'oléum sulfurique à 20 % de SO₃ contenant 250 mg de HgSO₄. Après avoir laissé revenir à température ordinaire, le milieu est amené progressivement à 150°C et ensuite, maintenu à cette température durant 16 heures. Le milieu est versé sur un mélange (50:50) MeOH-acétone refroidi vers -50°C. Il se forme un précipité beige qui est recristallisé dans le même mélange MeOH-acétone.
Rdt : (35-40 %)
P.F. : 297-300°C.

### Stade B : (2,4-dichloropyrid-5-yl)sulfonylguanidine

En procédant comme dans la préparation 14, mais en partant du composé hydroxylé obtenu au stade précédent, on obtient le composé du titre.
P.F. : 202-205°C.

### PREPARATION 16 :

### (2,6-DICHLOROPYRID-3-YL)SULFONYLGUANIDINE

### Stade A 3-amino-2,6-dichloropyridine

4 g de 2,6-dichloro-3-nitropyridine sont ajoutés à une suspension de 8 g de fer réduit dans 200 cm³ d'une solution hydroalcoolique (50:50) contenant 1,6 g de NH₄Cl. La suspension est portée à reflux durant 1 heure. Ensuite, l'insoluble est éliminé par filtration. Le filtrat est additionné de 0,4 g de Na₂SO₃ et de 0,4 g de NaH₂PO₂. La solution est ajustée à pH 8 et l'alcool est concentré sous dépression jusqu'à précipitation. Les cristaux correspondant à la 3-amino-2,6-dichloropyridine sont recueillis sur filtre, lavés a l'eau et séchés.
Rdt : 70 %
P.F. : 121-123°C.

### Stade B : (2,6-dichloropyrid-3-yl)sulfonylguanidine

A partir de 10 g de 3-amino-2,6-dichloropyridine, on prépare le chlorure de (2,6-dichloropyrid-3-yl)sulfonyle. Ce dernier est traité dans les mêmes conditions que lors de la préparation 14 en présence de 8,5 g de carbonate de guanidine.
Rdt : 30-35 %
P.F. : 245-250°C.

### PREPARATION 17 :

### 4-AMINO-3-CYCLOPENTYLCARBONYLSULFONAMIDOPYRIDINE

### Stade A : 4-cyclopentylcarboxamido-3-cyclopentylcarbonylsulfonamidopyridine

Le mélange de 1 g de (4-aminopyrid-3-yl)sulfonamide (préparation 2), 2 cm³ d'acide cyclopentane carboxylique et 8 cm³ d'oxychlorure de phosphore est porté à reflux pendant 10 minutes. Ensuite, le mileu réactionnel est concentré sous dépression (rotavapor). Le résidu huileux obtenu est trituré avec 100 cm³ d'eau glacée jusqu'à l'obtention d'un précipité blanc finement dispersé. Le précipité est recueilli sur filtre, lavé à l'eau et séché. Il est purifié par dissolution dans un minimum de méthanol chaud, décoloration au charbon absorbant, filtration et adjonction au filtrat de 2 volumes d'eau. Après une nuit à + 4°C, les cristaux sont recueillis sur filtre, lavés à l'eau et séchés.
Rdt : 80-85 %
P.F. : 219-222°C.

### Stade B : 4-amino-3-cyclopentylcarbonylsulfonamidopyridine

1 g de 4-cyclopentylcarboxamido-3-cyclopentylcarbonylsulfonamidopyridine obtenu au stade précédent est dissous dans 20 cm³ d'eau contenant 0,22 g de NaOH (2 équivalents). La solution est portée à reflux pendant 2 heures. La solution chaude obtenue est décolorée au charbon absorbant puis filtrée. Le filtrat refroidi est ajusté à pH 6 par HCl 0,1N. Le précipité obtenu est recueilli sur filtre, lavé à l'eau et séché.
Rdt : 80-85 %
P.F. : 215-217°C.

### PREPARATION 18 :

### 4-AMINO-3-TRIFLUOROACETYLSULFONAMIDOPYRIDINE

Le mélange de 1 g de (4-aminopyrid-3-yl)sulfonamide (préparation 2) et de 7,5 cm³ d'anhydride trifluoroacétique est porté à reflux pendant 4 heures. Après refroidissement, le précipité est filtré et lavé au diéthyléther. Le précipité est remis en suspension dans 25 cm³ de méthanol bouillant pendant 15 minutes. Après refroidissement, l'insoluble correspondant au composé du titre est recueilli sur filtre, lavé au méthanol, puis au diéthyléther, et séché.
Rdt : 80-85 %
P.F. : 262-266°C.

### PREPARATION 19 :

### 4-AMINO-5-ACETYLSULFONAMIDO-2-CHLOROPYRIDINE

### Stade A : (4-amino-2-chloropyrid-5-yl)sulfonamide

Une solution de 1 g de 3-amino-6-chloro-4H-pyrido[4,3-e] [1,2,4]thiadiazine 1,1-dioxyde monohydrate (composé obtenu à l'exemple 31 ci-après) dans 30 cm³ de H₂SO₄ 50 % v/v dans l'eau est portée à reflux pendant 15 minutes. Après refroidissement, la solution est allongée avec 300 cm³ d'eau glacée, puis neutralisée à pH 7 à l'aide de NaHCO₃ solide. Le précipité blanc cristallin obtenu est recueilli sur filtre, lavé à l'eau et séché.
Rdt : 80 %
P.F. : 240-242°C.

### Stade B : 4-acétylamino-5-acétylsulfonamido-2-chloropyridine

Le mélange de 1 g de (4-amino-2-chloropyrid-5-yl)sulfonamide (obtenu au stade précédent) et de 10 cm³ d'anhydride acétique est porté à reflux pendant 15 minutes. Après refroidissement, les cristaux sont recueillis sur filtre, lavés à l'anhydride acétique, puis au diéthyléther, et séchés.
Rdt : 85-90 %
P.F. : 219-224°C.

### Stade C : 4-amino-5-acétylsulfonamido-2-chloropyridine

La solution de 0,5 g de 4-acétylamino-5-acétylsulfonamido-2-chloropyridine (stade précédent) dans 10 cm³ d'eau contenant 0,137 g de NaOH (2 équivalents) est portée à reflux pendant 15 minutes. Après refroidissement, la solution est ajustée à pH 6 et le précipité obtenu est recueilli sur filtre, lavé à l'eau et séché.
Rdt : 50-55 %
P.F. : 235-238°C

### PREPARATION 20 :

### (2-AMINO-5-CHLOROPYRID-3-YL)SULFONAMIDE

2 g de 2-amino-5-chloropyridine sont dissous dans 10 cm³ d'acide chlorosulfonique maintenus à -5°C (bain de glace + sel). Le milieu réactionnel est amené progressivement à température ambiante, puis porté à reflux pendant 5 heures. Après refroidissement, la solution est versée sur de la glace, et le précipité de chlorure de 2-amino-5-chloropyrid-3-yl)sulfonyle est filtré et lavé avec un peu d'eau froide. Le précipité est dispersé sous forte agitation dans 50 cm³ d'ammoniaque (10 %). La suspension de composé du titre obtenue est alors concentrée sous vide jusqu'à un volume de 20 cm³ et le précipité cristallin est recueilli sur filtre, lavé à l'eau et séché.
Rdt : 45-50 %
P.F. : 215-220°C

### PREPARATION 21 :

### (4-ETHYLAMINOPYRID-3-YL)SULFONAMIDE

En procédant comme dans la préparation 13, mais en partant de 2 g de (4-chloropyrid-3-yl)sulfonamide et de 20 cm³ d'éthylamine à 70 % dans l'eau, on obtient le composé du titre.
Rdt : 65-70 %
P.F. : 192-193°C

### PREPARATION 22 :

### (4-PROPYLAMINOPYRID-3-YL)SULFONAMIDE

2 g de (4-chloropyrid-3-yl)sulfonamide sont dissous dans 10 cm³ de n-propylamine et 10 cm³ d'isopropanol. Le mélange est porté à reflux pendant 2 heures, puis la solution est refroidie et le solvant est évaporé. Le solide obtenu est redispersé dans l'eau (20 cm³). L'insoluble est recueilli sur le filtre, lavé à l'eau et séché.
Rdt : 90-95 %
P.F. : 180-181°C

### PREPARATION 23 :

### (4-BUTYLAMINOPYRID-3-YL)SULFONAMIDE

Le composé est obtenu selon la technique décrite pour la préparation 22 en utilisant la n-butylamine.
Rdt : 80-85 %
P.F. : 143-145°C

### PREPARATION 24 :

### [4-(2',2',2'-TRIFLUOROETHYLAMINO)PYRID-3-YL]SULFONAMIDE

Le composé est obtenu selon la technique décrite pour la préparation 13 en utilisant la trifluoroéthylamine à 50 % v/v dans l'eau.
Rdt : 35-40 %
P.F. : 221-222°C

### PREPARATION 25 : (3-AMINO-5-METHYLPYRID-2-YL)SULFONAMIDE

### Stade A : 2-hydroxy-5-méthyl-3-nitropyridine

Dans un ballon de 600 cm³ sont dissous 25g de 2-amino-5-méthylpyridine dans 50 cm³ d'acide sulfurique concentré. Un mélange constitué de 40 cm³ d'acide nitrique concentré et 40 cm³ d'acide sulfurique concentré est ajouté progressivement. Le milieu réactionnel s'échauffe avec formation de mousse. La température est maintenue constante à 130°C pendant toute la durée de l'addition de l'acide. La solution colorée est ensuite versée sur 300 g de glace, et le pH est ramené vers 3-4 par addition d'ammoniaque. Après repos au réfrigérateur, le précipité obtenu est recueilli sur filtre, lavé à l'eau et séché. Il est recristallisé dans l'eau chaude.
Rdt : 35-40%
P.F : 243-246°C

### Stade B : 2-chloro-5-méthyl-3-nitropyridine

5 g de 2-hydroxy-5-méthyl-3-nitropyridine sont dissous dans 32 cm³ de chlorure de thionyle additionnés de 2 cm³ de N,N-diméthylformamide. Le mélange est ensuite porté à reflux pendant 2 heures. Après élimination du solvant sous dépression, le produit est partagé entre l'eau distillée (30cm³) et le chloroforme (3x100 cm³). Les fractions chloroformiques sont rassemblées, séchées sur MgSO₄, filtrées, puis concentrées sous vide. Le résidu est recristallisé dans éthanol/eau.
Rdt : 60-65%
P.F : 48°C

### Stade C : chlorhydrate de 2-(5-méthyl-3-nitropyrid-2-yl)-2-isothiourée

La solution de 23 g de 2-chloro-5-méthyl-3-nitropyridine et de 11,14 g de thiourée dans 130 cm³ d'éthanol est portée à reflux pendant 2 heures. Après refroidissement et addition de 20 cm³ d'éther de pétrole (40-60°C), le précipité obtenu est recueilli sur filtre, lavé à l'éther de pétrole et séché.
Rdt : 70-75 %
P.F : 205-206°C

### Stade D : 2-mercapto-5-méthyl-3-nitropyridine

La solution de 19 g de sel de thiouronium dans 110 cm³ d'eau est additionnée sous agitation de 10,05 g de carbonate sodique, puis d'une solution de 7,59 g de NaOH dans 10 cm³ de HCl 1N. Le précipité obtenu est recueilli sur filtre, lavé à l'eau et séché.
Rdt : 75-80%
P.F: 198-200°C

### Stade E : (5-méthyl-3-nitropyrid-2-y)sulfénamide

La solution de 12 g de 2-mercapto-5-méthyl-3-nitropyridine et 28,6 g de triéthylamine dans 180 cm³ de dichlorométhane est additionnée goutte à goutte de 12 g d'acide hydroxylamine-O-sulfonique dissous dans un minimum d'eau. A la fin de la réaction, le solvant est éliminé sous dépression, et le résidu est repris par 20 cm³ d'eau. Le précipité obtenu est recueilli sur filtre, lavé à l'eau et séché. Il est recristallisé dans le mélange dichlorométhane-pétroléine.
Rdt : 90-95%
P.F : 143-145°C

### Stade F : (5-méthyl-3-nitropyrid-2-yl)sulfonamide

La solution de 6 g de (5-méthyl-3-nitropyrid-2-yl)sulfénamide dans 150 cm³ d'acétonitrile est additionnée progressivement de 2,5 g de permanganate de potassium dissous dans un minimum d'eau. Le terme de la réaction est apprécié par chromatographie couche mince. Le précipité noir est éliminé par filtration, et le filtrat est concentré jusqu'à petit volume. La solution est ajustée à pH 3, et le produit est abandonné à la cristallisation à 4°C pendant 6 heures. Les cristaux sont recueillis sur filtre, lavés à l'eau et séchés.
Rdt : 40-50 %
P.F : 160-162°C

### Stade G : (3-amino-5-méthylpyrid-2-yl)sulfonamide

A la solution de 2,17 g de (5-méthyl-3-nitropyrid-2-yl)sulfonamide dans 60 cm³ de solution hydroalcoolique (EtOH-H₂O : 1-1) est ajouté 3,46 g de fer réduit et 0,53 g de chlorure ammonique. Le mélange est porté à reflux pendant 20 minutes et ensuite filtré à chaud. Le filtrat est concentré jusqu'à petit volume et abandonné à 4°C pendant 12 heures. Le précipité cristallin obtenu est recueilli sur filtre, lavé à l'eau et séché.
Rdt :95-100 %
P.F :192-193°C
Les exemples suivants illustrent l'invention mais ne la limitent en aucune façon.

### EXEMPLE 1 : 2,3-DIHYDRO-4H-PYRIDO[3,2-e][1,2,4]THIADIAZINE 1,1-DIOXYDE

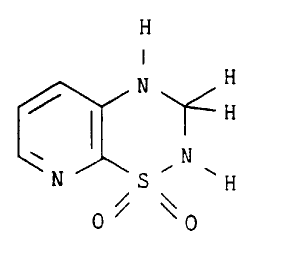

Le mélange de 1 g de (3-aminopyrid-2-yl)sulfonamide (préparation 1) et de 0,23 g de paraformaldéhyde (1,3 éq.) dans 10 cm³ d'isopropanol additionné de 10 gouttes d'acétate d'éthyle saturé de HCl gazeux est porté à reflux pendant 1 à 2 heures. Après refroidissement, le précipité cristallin du composé du titre est recueilli sur filtre et lavé à l'isopropanol. Le produit est recristallisé dans l'eau chaude.
Rdt : 90 %
P.F. : 242-246°C.

### EXEMPLE 2 : 2,3-DIHYDRO-4H-PYRIDO[4,3-e][1,2,4]THIADIAZINE 1,1-DIOXYDE

En procédant comme dans l'exemple 1 (excepté la durée du reflux qui est de 2 à 4 heures), mais en remplaçant la (3-aminopyrid-2-yl)sulfonamide par la (4-aminopyrid-3-yl) sulfonamide (préparation 2), on obtient le composé du titre.
Rdt : 70-75 %
P.F. (monohydrate) : 245-248°C.

### EXEMPLE 3 : 4-ISOPROPYL-2,3-DIHYDRO-4H-PYRIDO[4,3-e][1,2,4]THIADIAZINE 1,1-DIOXYDE

Le mélange de 1 g de (4-isopropylaminopyrid-3-yl)sulfonamide (préparation 3) et de 1 g de paraformaldéhyde dans 20 cm³ d'isopropanol additionné de 10 gouttes d'acétate d'éthyle saturé de HCl gazeux est porté à reflux pendant 3 heures. Après refroidissement, le précipité cristallin est filtré et lavé à l'isopropanol. Le précipité est dissous dans 150 cm³ de méthanol chaud. L'insoluble éventuel est filtré. Le filtrat est concentré à siccité et le résidu est repris par 30 cm³ d'eau. La suspension aqueuse est ajustée à pH 7-7,5 (NaHCO₃) et le précipité correspondant au composé du titre est filtré, lavé à l'eau et séché.
Rdt : 80-85 %
P.F. : 202-203°C.

### EXEMPLE 4 : 4-CYCLOHEXYL-2,3-DIHYDRO-4H-PYRIDO[4,3-e][1,2,4]THIADIAZINE 1,1-DIOXYDE

En procédant comme dans l'exemple 3 mais en remplaçant la préparation 3 par la préparation 4, on obtient le composé du titre.
Rdt : 75-80 %
P.F. : 247-250°C.

### EXEMPLE 5 : 4-METHYL-2,3-DIHYDRO-4H-PYRIDO[4,3-e][1,2,4]THIADIAZINE 1,1-DIOXYDE

Un mélange de 1 g de (4-méthylaminopyrid-3-yl)sulfonamide (préparation 6) et de 1 g de paraformaldéhyde (excès) dans 10 cm³ d'isopropanol additionné de 4 cm³ d'acétate d'éthyle saturé de HCl gazeux est porté à reflux pendant 10 heures. Après ce laps de temps, le solvant est éliminé sous dépression (rotavapor) et le résidu est redissous dans le méthanol. Un insoluble éventuel est filtré, et le filtrat, additionné de 2 volumes de diéthyléther laisse précipiter le composé du titre sous forme de cristaux. Les cristaux sont recueillis sur filtre, lavés au diéthyléther et séchés.
Rdt : 60-65 %
P.F. (chlorhydrate) : 291-294°C.

### EXEMPLE 6 : 4-CYCLOOCTYL-2,3-DIHYDRO-4H-PYRIDO[4,3-e][1,2,4]THIADIAZINE 1,1-DIOXYDE

En procédant comme dans l'exemple 5, mais en remplaçant la préparation 6 par la préparation 5, on obtient le composé du titre.
Rdt : 65-70 %
P.F. (chlorhydrate) : 254-255°C.

### EXEMPLE 7 : 2-ISOPROPYL-2,3-DIHYDRO-4H-PYRIDO[4,3-e][1,2,4]THIADIAZINE 1,1-DIOXYDE

Le mélange de 0,5 g de N-isopropyl-(4-aminopyrid-3-yl)sulfonamide (préparation 7) et de 0,5 g de paraformaldéhyde dans 15 cm³ d'isopropanol additionné de 50 gouttes d'acétate d'éthyle saturé de HCl gazeux est porté à reflux pendant 24 heures. Après ce laps de temps, le solvant est éliminé sous dépression. Le résidu est redissous dans 20 cm³ de méthanol. L'insoluble éventuel est éliminé par filtration. Le filtrat, additionné de 40 cm³ d'eau et conservé 2 heures à + 4°C abandonne un précipité de dérivé isopropoxyméthyléné de la préparation 7. Il est recueilli sur filtre, lavé à l'eau, séché, et recristallisé dans le mélange CHCl₃/éther de pétrole (40-60°C) (1:2). Le produit sec est introduit dans un ballon ouvert et amené à la température de 180-190°C. Après 15 à 30 minutes, la masse fondue se solidifie. Elle est alors refroidie, puis dissoute dans un petit volume de chloroforme. L'adjonction d'un volume d'éther de pétrole (40-60°C) provoque la précipitation de cristaux du composé du titre. Ils sont recueillis sur filtre, lavés à l'éther de pétrole (40-60°C) et séchés.
Rdt : 25-30 %
P.F. : 209-213°C.

### EXEMPLE 8 : 4-METHYL-2,3-DIHYDRO-4H-PYRIDO[2,3-e][1,2,4]THIADIAZINE 1,1-DIOXYDE

En procédant comme dans l'exemple 1, mais en utilisant seulement 0,16 g de paraformaldéhyde, en maintenant le reflux pendant 24 heures et en recristallisant dans un mélange MeOH/H₂O (1:3), on obtient, en remplaçant la préparation 1 par la préparation 8, le composé du titre.
Rdt : 75-80 %
P.F. : 184-187°C.

### EXEMPLE 9 : 2-PENTYL-2,3-DIHYDRO-4H-PYRIDO[4,3-e][1,2,4]THIADIAZINE 1,1-DIOXYDE

Le mélange de 0,5 g de N-pentyl-(4-aminopyrid-3-yl)sulfonamide (préparation 9) et de 0,5 g de paraformaldéhyde dans 15 cm³ d'isopropanol additionné de 50 gouttes d'acétate d'éthyle saturé de HCl gazeux est porté à reflux pendant 48 heures. Après ce laps de temps, le solvant est éliminé sous dépression. Le résidu est suspendu dans 20 cm³ de NaOH 0,1N. Après 1 heure d'agitation à température ambiante, l'insoluble est recueilli sur filtre, lavé à l'eau et recristallisé deux fois dans le mélange méthanol/eau (1:2), puis une fois dans CHCl₃/éther de pétrole (40-60°C) (1:1).
Rdt : 30-35 %
P.F. : 188-192°C.

### EXEMPLE 10 : 2-CYCLOHEXYL-2,3-DIHYDRO-4H-PYRIDO[4,3-e][1,2,4]THIADIAZINE 1,1-DIOXYDE

En procédant comme dans l'exemple 7 mais en remplaçant la préparation 7 par la préparation 10, on obtient le composé du titre.
P.F. : 173°C.

### EXEMPLE 11 : 2-PHENYL-2,3-DIHYDRO-4H-PYRIDO[4,3-e][1,2,4]THIADIAZINE 1,1-DIOXYDE

En procédant comme dans l'exemple 9, mais en remplaçant la préparation 9 par la préparation 11, on obtient le produit du titre.
Rdt : 30-35 %
P.F. : 225-230°C.

### EXEMPLE 12 : 3-METHYL-2,3-DIHYDRO-4H-PYRIDO[3,2-e][1,2,4]THIADIAZINE 1,1-DIOXYDE

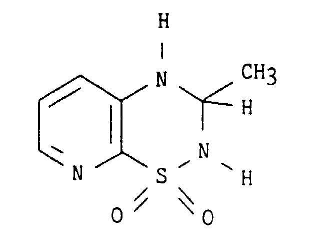

Le mélange de 1 g de (3-aminopyrid-2-yl)sulfonamide (préparation 1) et de 0,28 g d'acétaldéhyde dans 10 cm³ d'isopropanol additionné de 10 gouttes d'acétate d'éthyle saturé en HCl gazeux est porté à reflux pendant 1 à 2 heures. Après refroidissement, le précipité cristallin du composé du titre est recueilli sur filtre et lavé à l'isopropanol. Le 3-méthyl-2,3-dihydro-4H-pyrido[3,2-e][1,2,4]thiadiazine 1,1-dioxyde est recristallisé dans le mélange CH₃OH:H₂O (1:4).
Rdt : 90 **%**
P.F. : 204-209°C.

### EXEMPLE 13 : 3-METHYL-2,3-DIHYDRO-4H-PYRIDO[4,3-e][1,2,4]THIADIAZINE 1,1-DIOXYDE

En procédant comme dans l'exemple 12, mais en remplaçant la préparation 1 par la préparation 2, on obtient le composé du titre.
Rdt : 70-75 %
P.F. (monohydrate) : 229-231°C.

### EXEMPLE 14 : 2,3-DIMETHYL-2,3-DIHYDRO-4H-PYRIDO[4,3-e][1,2,4]THIADIAZINE 1,1-DIOXYDE

1 g de N-méthyl-(4-aminopyrid-3-yl)sulfonamide (préparation 12) est porté à reflux pendant 6 heures dans le mélange de 8 cm³ d'isopropanol, 32 cm³ d'acétaldéhyde et 6 cm³ d'acétate d'éthyle saturé en HCl gazeux. Après ce laps de temps, le milieu réactionnel est concentré sous dépression (rotavapor). La masse huileuse obtenue est dissoute et ajustée à pH 7 par du NaHCO₃. La suspension aqueuse est extraite 3 fois par 100 cm³ de dichlorométhane. La phase organique est séchée (MgSO₄), filtrée et concentrée à siccité. le résidu obtenu est dissous dans 10 cm³ de méthanol, puis additionné de 10 cm³ d'acétate d'éthyle saturé en HCl gazeux. L'adjonction de 60 cm³ de diéthyléther provoque la précipitation de cristaux correspondant au composé du titre. Ils sont recueillis sur filtre, lavés au diéthyléther et séchés.
Rdt : 60-65 %
P.F. (chlorhydrate) : 252-255°C.

### EXEMPLE 15 : 2,3-DIHYDRO-4H-PYRIDO[2,3-e][1,2,4]THIADIAZINE 1,1-DIOXYDE

### Stade A : 4H-pyrido[2,3-e][1,2,4]thiadiazine 1,1-dioxyde

Le mélange de 2 g de (2-aminopyrid-3-yl)sulfonamide (préparation 6) et de 20 cm³ d'orthoformiate d'éthyle est porté à reflux pendant 1 heure. Après refroidissement, le précipité cristallin obtenu est recueilli sur filtre et lavé à l'éther.
Rdt : 90-95 %
P.F. : 298-301°C.

### Stade B : 2,3-dihydro-4H-pyrido[2,3-e][1,2,4]thiadiazine 1,1-dioxyde

1 g de 4H-pyrido[2,3-e][1,2,4]thiadiazine 1,1-dioxyde est mis en suspension dans 30 cm³ d'eau. A cette suspension est ajoutée une solution de 0,83 g de NaBH₄ dans 5 cm³ d'eau. Après 15 minutes d'agitation à température ambiante, le pH de la solution est ajusté à 6,5-7 à l'aide de HCl dilué. Le précipité obtenu est filtré, lavé à l'eau et séché. Le composé sec est dissous dans 200 cm³ de dichlorométhane. L'insoluble éventuel est éliminé par filtration. Le filtrat est additionné d'un égal volume d'éther de pétrole (40-60°C) et placé à + 4° C pendant une nuit. Les cristaux obtenus sont recueillis sur filtre et séchés.
Rdt : 75-80 %
P.F. : 178-179°C.

### EXEMPLE 16 : 4H-PYRIDO[3,2-e][1,2,4]THIADIAZINE 1,1-DIOXYDE

En procédant comme au stade A de l'exemple 15, mais en utilisant la préparation 1, on obtient le composé du titre.
Rdt : 85-90 %
P.F. : 320-325°C.

### EXEMPLE 17 : 3-METHYL-4H-PYRIDO[3,2-e][1,2,4]THIADIAZINE 1,1-DIOXYDE

1g de (3-aminopyrid-2-yl)sulfonamide (préparation 1) et 1 g d'acide p-toluènesulfonique sont mis en solution dans 6 cm³ d'orthoacétate d'éthyle. Après 10 minutes à température ambiante, le précipité obtenu est recueilli sur filtre, lavé au diéthyléther et séché. Il est recristallisé dans l'eau chaude.
Rdt : 80-85 %
P.F. : 263-266°C.

### EXEMPLE 18 : 4H-PYRIDO[4,3-e][1,2,4]THIADIAZINE 1,1-DIOXYDE

3,3 cm³ d'acide formique et 6,6 cm³ d'anhydride acétique sont maintenus sous agitation à 50°C pendant 15 minutes. A cette solution est ajoutée 1 g de (4-aminopyrid-3-yl)sulfonamide (préparation 2) et le milieu réactionnel est porté à reflux pendant 2 heures. Après refroidissement, les cristaux obtenus sont recueillis sur filtre, lavés avec un peu d'acide acétique, puis avec du diéthyléther, et séchés.
Rdt : 75-80 %
P.F. : 296-298°C.

### EXEMPLE 19 : 2-ISOPROPYL-2H-PYRIDO[4,3-e][1,2,4]THIADIAZINE 1,1-DIOXYDE

Un mélange de 0,5 g de N-isopropyl-(4-aminopyrid-3-yl)sulfonamide (préparation 7) et de 5 cm³ d'orthoformiate d'éthyle est maintenu en vase ouvert à 120°C pendant 4 heures. Après concentration de la solution sous dépression, le résidu huileux obtenu est trituré avec 10 cm³ d'eau glacée. Le précipité cristallin qui apparaît est recueilli sur filtre et lavé à l'eau. Il est recristallisé dans le mélange méthanol : eau (1:2).
Rdt : 80-85 %
P.F. : 175-178°C.

### EXEMPLE 20 : 3-METHYL-4H-PYRIDO[4,3-e][1,2,4]THIADIAZINE 1,1-DIOXYDE

1 g de (4-aminopyrid-3-yl)sulfonamide (préparation 2) est maintenu pendant 4-6 heures à reflux dans 10 cm³ d'anhydride acétique. Après refroidissement, le milieu réactionnel est additionné de 60 cm³ de diéthyléther. Le précipité obtenu est filtré, lavé au diéthyléther et séché. Il est recristallisé dans l'eau chaude.
Rdt : 70-75 %
P.F. (monohydrate) : 264-268°C.

### EXEMPLE 21 : 3-ETHYL-4H-PYRIDO[4,3-e][1,2,4]THIADIAZINE 1,1-DIOXYDE

Le mélange de 1 g de (4-aminopyrid-3-yl)sulfonamide (préparation 2) et de 10 cm³ d'anhydride propionique est chauffé à 150°C pendant 8 heures. Après refroidissement, un égal volume de diéthyléther est ajouté et le précipité obtenu est recueilli sur filtre, lavé au diéthyléther et séché. Il est recristallisé dans l'eau chaude.
Rdt : 45-50 %
P.F. (monohydrate) : 220-223°C.

### EXEMPLE 22 : 3-PROPYL-4H-PYRIDO[4,3-e][1,2,4]THIADIAZINE 1,1-DIOXYDE MONOHYDRATE

Le mélange de 1 g de (4-aminopyrid-3-yl)sulfonamide (préparation 2) et de 10 cm³ d'anhydride butyrique est chauffé à 180°C pendant 18 heures. Après refroidissement, les cristaux obtenus sont recueillis sur filtre, lavés avec un peu d'anhydride butyrique, du diéthyléther, puis séchés. Le produit est recristallisé dans l'eau chaude.
Rdt : 50-55 %
P.F. (monohydrate) : 210-212°C.

### EXEMPLE 23 : 3-ISOPROPYL-4H-PYRIDO[4,3-e][1,2,4]THIADIAZINE 1,1-DIOXYDE

Le mélange de 1 g de (4-aminopyrid-3-yl)sulfonamide (préparation 2) et de 10 cm³ d'anhydride isobutyrique est maintenu à 170°C pendant 72 heures. Après refroidissement, le précipité obtenu est filtré, lavé au diéthyléther et séché. Le produit est dissous à chaud dans un minimum de méthanol. La solution chaude est décolorée au charbon absorbant, filtrée, et ensuite additionnée de 3 volumes d'eau. Après une nuit de repos à + 4°C, les cristaux obtenus sont recueillis sur filtre, lavés à l'eau et séchés.
Rdt : 40-45 %
P.F. : 248-250°C.

### EXEMPLE 24 : 3-TERT BUTYL-4H-PYRIDO[4,3-e][1,2,4]THIADIAZINE 1,1-DIOXYDE

Le composé du titre est obtenu à partir de (4-aminopyrid-3-yl)sulfonamide (préparation 2) et d'anhydride triméthylacétique en appliquant les conditions expérimentales décrites pour l'exemple 23.
Rdt : 40-45 %
P.F. : 290-293°C.

### EXEMPLE 25 : 3,4-DIMETHYL-4H-PYRIDO[4,3-e][1,2,4]THIADIAZINE 1,1-DIOXYDE

Le mélange de 1 g de (4-méthylaminopyrid-3-yl)sulfonamide (préparation 13) et de 10 cm³ d'anhydride acétique est porté à reflux pendant 4 heures. Après refroidissement, le milieu réactionnel est additionné d'un égal volume de diéthyléther sous agitation. Le précipité cristallin est filtré, lavé au diéthyléther et séché. Il est purifié par dissolution dans le minimum de méthanol chaud et adjonction de 2 volumes d'eau. Après repos à + 4°C, les cristaux obtenus sont recueillis sur filtre, lavés à l'eau et séchés.
Rdt : 80-85 % P.F. : 227-228°C.

### EXEMPLE 26 : 4-ISOPROPYL-3-METHYL-4H-PYRIDO[4,3-e][1,2,4]THIADIAZINE 1,1-DIOXYDE

Le composé du titre est obtenu à partir de (4-isopropylaminopyrid-3-yl)sulfonamide (préparation 3) en appliquant les conditions expérimentales décrites pour l'exemple 25.
Rdt : 70 %
P.F. : 196-197°C.

### EXEMPLE 27 : 4-CYCLOHEXYL-3-METHYL-4H-PYRIDO[4,3-e][1,2,4]THIADIAZINE 1,1-DIOXYDE

Le composé du titre est obtenu à partir de (4-cyclohexylaminopyrid-3-yl)sulfonamide (préparation 4) en appliquant les conditions expérimentales décrites pour l'exemple 25.
Rdt : 60-65 %
P.F. : 224-226°C.

### EXEMPLE 28 : 4-CYCLOOCTYL-3-METHYL-4H-PYRIDO[4,3-e][1,2,4]THIADIAZINE 1,1-DIOXYDE

Le composé du titre est obtenu à partir de (4-cyclooctylaminopyrid-3-yl)sulfonamide (préparation 5) en appliquant les conditions expérimentales décrites pour l'exemple 25.
Rdt : 60-65 %
P.F. : 207-210°C.

### EXEMPLE 29 : 2-CYCLOHEXYL-2H-PYRIDO[4,3-e][1,2,4]THIADIAZINE 1,1-DIOXYDE

Le composé du titre est obtenu à partir de N-cyclohexyl-(4-aminopyrid-3-yl)sulfonamide (préparation 10) en appliquant les conditions expérimentales décrites pour l'exemple 19.
Rdt : 80-85 %
P.F. : 144-147°C.

### EXEMPLE 30 : 3-AMINO-4H-PYRIDO[4,3-e][1,2,4]THIADIAZINE 1,1-DIOXYDE

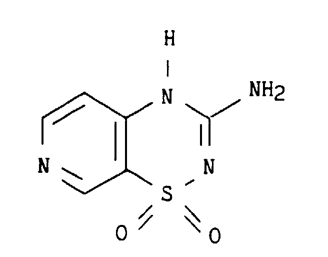

Le mélange de 0,25 g de (4-chloropyrid-3-yl)sulfonylguanidine (préparation 14) et de 0,25 g de carbonate de potassium dans 5 cm³ de dioxane-DMF (80:20) est chauffé à reflux pendant 24 heures.
Après refroidissement, le précipité blanc obtenu est filtré, puis redissous dans un minimum d'eau. La solution est ajustée à pH 7 et le précipité blanc correspondant au composé du titre est recueilli sur filtre, lavé à l'eau et séché.
Rdt : 40-45 %
P.F. (monohydrate) : 327-330°C.

### EXEMPLE 31 : 3-AMINO-6-CHLORO-4H-PYRIDO[4,3-e][1,2,4]THIADIAZINE 1,1-DIOXYDE

En procédant comme dans l'exemple 30, mais en partant de la préparation 15, on obtient le composé du titre.
Rdt : 40-45 %
P.F. (monohydrate) : 316-319°C.

### EXEMPLE 32 : 3-AMINO-6-CHLORO-4H-PYRIDO[2,3-e][1,2,4]THIADIAZINE 1,1-DIOXYDE

Le composé du titre est obtenu à partir de la préparation 16 selon la technique décrite pour l'exemple 30.
Rdt : 75 %
P.F. (monohydrate) : 327-330°C.

### EXEMPLE 33 : 3-CYCLOPENTYL-4H-PYRIDO[4,3-e][1,2,4]THIADIAZINE 1,1-DIOXYDE

La solution de 1 g de 4-amino-3-cyclopentylcarbonylsulfonamidopyridine (préparation 17), dans 1 cm³ de DMF et 10 cm³ d'oxychlorure de phosphore est agitée à température ambiante pendant 3 heures. Les solvants sont éliminés sous dépression (rotavapor) et le résidu est dissous dans 10 cm³ d'eau. La solution est ajustée à pH 4 (NaHCO₃) et le précipité cristallin obtenu est recueilli sur filtre, lavé à l'eau et séché.
Rdt : 15-20 %
P.F. : 263-266°C.

### EXEMPLE 34 : 3-TRIFLUOROMETHYL-4H-PYRIDO[4,3-e][1,2,4]THIADIAZINE 1,1-DIOXYDE

Le mélange de 1 g de 4-amino-3-trifluoroacétylsulfonamidopyridine (préparation 18) et de 20 cm³ d'oxychlorure de phosphore est porté à reflux pendant 18 heures. Le solvant est éliminé sous dépression et l'huile obtenue, triturée avec 5 ml d'eau glacée, laisse apparaître un précipité. Celui-ci est recueilli sur filtre, lavé à l'eau et séché. Il est ensuite purifié par dissolution dans un minimum de méthanol, puis adjonction de 3 volumes de diéthyléther et de 3 volumes d'éther de pétrole (40-60°). Les cristaux obtenus sont recueillis sur filtre, lavés au diéthyléther et séchés.
Rdt : 60-65 %
P.F. (monohydrate) : 236-240°C.

### EXEMPLE 35 : 6-CHLORO-3-METHYL-4H-PYRIDO[4,3-e][1,2,4]THIADIAZINE 1,1-DIOXYDE

Dans un ballon en vase ouvert placé sur un bain d'huile à 240°C, est introduit 0,2 g de 4-amino-5-acétylsulfonamido-2-chloropyridine (préparation 19). La température du bain d'huile est amenée en l'espace d'une demi-heure de 240°C à 270°C. La masse fondue est ensuite refroidie. Elle est dissoute dans 4 cm³ d'une solution tiède de NaHCO₃ (2 % m/v). La solution est décolorée au charbon absorbant, filtrée, puis ajustée à pH 4 à l'aide d'acide formique. Le précipité cristallin obtenu est recueilli sur filtre, lavé à l'eau et séché.
Rdt : 40 %
P.F. (monohydrate) : 309-312°C.

### EXEMPLE 36 : 7-CHLORO-3-METHYL-4H-PYRIDO[2,3-e][1,2,4]THIADIAZINE 1,1-DIOXYDE

Le mélange de 1 g de (2-amino-5-chloropyrid-3-yl)sulfonamide (préparation 20) et de 10 cm³ d'anhydride acétique est porté à reflux pendant 6 heures. Après refroidissement, les cristaux sont filtrés, lavés avec un peu d'anhydride acétique, puis du diéthyléther, et séchés, Les cristaux sont redissous dans 50 cm³ de NaOH 0,1N. L'insoluble éventuel est éliminé par filtration. Le filtrat, ajusté à pH 4 à l'aide d'acide formique laisse précipiter les cristaux. Ils sont recueillis sur filtre, lavés à l'eau et séchés.
Rdt : 60-65 %
P.F. : > 340°C.

### EXEMPLE 37 : 3-ISOPROPOXY-4H-PYRIDO[3,2-e][1,2,4]THIADIAZINE 1,1-DIOXYDE

### Stade A : 3-oxo-2,3-dihydro-4H-pyrido[3,2-e][1,2,4]thiadiazine 1,1-dioxyde

Le mélange intime de 5 g de (3-aminopyrid-2-yl)sulfonamide (préparation 1) et de 1,91 g d'urée est amené progressivement à la température de 200°C (fusion). Après cessation de dégagement gazeux et solidification de la masse, le milieu réactionnel est refroidi à température ambiante. La masse solide est redissoute dans du NaOH 1N, et la solution obtenue, éventuellement décolorée au charbon absorbant, est alors ajustée à pH 2 à l'aide de HCl 1N. Le précipité blanc correspondant au composé du titre est recueilli sur filtre, lavé à l'eau et séché.
Rdt : 85-90 %
P.F. : > 300°C.

### Stade B : 3-isopropoxy-4H-pyrido[3,2-e][1,2,4]thiadiazine 1,1-dioxyde

Le sel sodique du 3-oxo-2,3-dihydro-4H-pyrido[3,2-e] [1,2,4] thiadiazine 1,1-dioxyde (0,5 g) obtenu au stade précédent, est préparé en solution méthanolique (7 cm³) par l'action de 1,1 équivalent de NaOH (0,11 g). Après élimination du solvant sous dépression, le sel solide est redissous dans 5 cm³ de DMF, puis additionné de 0,4 cm³ d'iodure d'isopropyle. Après 7 heures de réaction à température ambiante, le solvant est éliminé sous dépression, et le résidu est repris par 15 cm³ d'eau. La suspension aqueuse est ajustée à pH 12 avec du NaOH 2N. L'insoluble, constitué de 2-isopropyl-3-oxo-2,3-dihydro-4H-pyrido[3,2-e][1,2,4]thiadiazine 1,1-dioxyde (P.F. : 218-221°C), est éliminé par filtration. Le filtrat est ajusté à pH 2 à l'aide de HCl 1N. Le précipité correspondant au composé du titre est recueilli sur filtre, lavé à l'eau et séché.
P.F. : 196-199°C.

### EXEMPLE 38 : 3-THIOXO-2,3-DIHYDRO-4H-PYRIDO[4,3-e][1,2,4]THIADIAZINE 1,1-DIOXYDE

### Stade A : 3-oxo-2,3-dihydro-4H-pyrido[4,3-e][1,2,4]thiadiazine 1,1-dioxyde

Le composé du titre est obtenu à partir de (4-aminopyrid-3-yl)sulfonamide (préparation 2) en appliquant les conditions expérimentales décrites pour l'exemple 37.
Rdt : 85-90 %
P.F. (monohydrate) : > 330°C.

### Stade B : 3-thioxo-2,3-dihydro-4H-pyrido[4,3e][1,2,4]thiadiazine 1,1-dioxyde

Le mélange de 3 g de 3-oxo-2,3-dihydro-4H-pyrido[4,3-e][1,2,4] thiadiazine 1,1-dioxyde obtenu au stade précédent et de 5,022 g de P₂S₅ dans 30 cm³ de pyridine anhydre est porté à reflux pendant 24 heures. Le milieu réactionnel est ensuite concentré sous dépression (rotavapor). Le résidu est dissous par un minimum de NaOH 2N. La solution obtenue est traitée au charbon actif, filtrée, puis ajustée à pH 2 à l'aide de HCl 1N. Le précipité est recueilli sur filtre et lavé à l'H₂O. Il est redissous dans une solution aqueuse de NaHCO₃ (2,5 %, m/v), traité au charbon actif, et reprécipité à pH 2 à l'aide de HCl 1N.
Rdt : 45-50 %
P.F. (monohydrate) : 292-294°C.

### EXEMPLE 39 : 3-METHYLTHIO-4H-PYRIDO[4,3-e][1,2,4]THIADIAZINE 1,1-DIOXYDE

### Premier procédé :

5,5 g de 3-thioxo-2,3-dihydro-4H-pyrido[4,3-e][1,2,4]thiadiazine 1,1-dioxyde (obtenu à l'exemple 38) sont mis en solution dans 165 cm³ d'eau contenant 4 g de NaHCO₃. A cette solution sont ajoutés 120 cm³ de méthanol, puis 8 cm³ d'iodure de méthyle. Après 1 heure d'agitation à température ambiante, le milieu réactionnel est concentré à un volume de 150 cm³ (rotavapor), puis ajusté à pH 2-3. Le précipité obtenu est recueilli sur filtre, lavé à l'eau et séché.
Rdt : 65-70 %
P.F. (monohydrate) : 242-245°C.

### Second procédé :

Une solution de 1 g de 3-thioxo-2,3-dihydro-4H-pyrido[4,3-e] [1,2,4]thiadiazine 1,1-dioxyde dans 10 cm³ de DMF anhydre est additionnée de 1,2 cm³ de sulfate de diméthyle. Après plusieurs heures à température ambiante, le solvant est éliminé sous dépression, et le résidu est redissous dans un minimum de NaOH 1N. La solution, décolorée au charbon absorbant et filtrée, est ajustée à pH 7 à l'aide de HCl 0,1N. Le précipité blanc correspondant au composé du titre est recueilli sur filtre, lavé à l'eau et séché.
Rdt : 80-85 %

### EXEMPLE 40 : 3-ISOPROPYLAMINO-4H-PYRIDO[4,3-e][1,2,4]THIADIAZINE 1,1-DIOXYDE

La solution de 0,7 g de 3-méthylthio-4H-pyrido[4,3-e] [1,2,4]thiadiazine 1,1-dioxyde obtenu à l'exemple 39 dans 7 cm³ d'isopropylamine est maintenue dans une bombe scellée à 150°C pendant 4 heures. Après refroidissement, la solution est concentrée à siccité. le résidu est repris par 10 cm³ d'eau, et le pH est ajusté à la valeur de 7. Le précipité obtenu est recueilli sur filtre, lavé à l'eau et recristallisé dans l'eau chaude.
Rdt : 75 %
P.F. (monohydrate) : 197-200°C.

### EXEMPLE 41 : 3-PROPYLAMINO-4H-PYRIDO-[4,3-e][1,2,4]THIADIAZINE 1,1-DIOXYDE MONOHYDRATE

En procédant comme dans l'exemple 40, mais en remplaçant l'isopropylamine par la propylamine, on obtient le composé du titre.
Rdt : 75-80 %
P.F. : 194-197°C.

### EXEMPLE 42 A 44 :

En procédant comme dans l'exemple 40, mais en remplaçant l'isopropylamine par l'amine appropriée, on obtient les composés des titres suivants :

### EXEMPLE 42 : 3-BUTYLAMINO-4H-PYRIDO[4,3-e][1,2,4]THIADIAZINE 1,1-DIOXYDE

Rdt : 80-85 %
P.F. : 167-170°C.

### EXEMPLE 43 : 3-(2-METHYLPROPYL)AMINO-4H-PYRIDO[4,3-e][1,2,4]THIADIAZINE 1,1-DIOXYDE

Rdt : 75-80 %
P.F. : 221-224°C.

### EXEMPLE 44 : 3-(1-METHYLPROPYL)AMINO-4H-PYRIDO[4,3-e][1,2,4]THIADIAZINE 1,1-DIOXYDE

Rdt : 75-80 %
P.F. (monohydrate) : 212-215°C.

### EXEMPLE 45 : 3-(1,2-DIMETHYLPROPYL)AMINO-4H-PYRIDO[4,3-e][1,2,4] THIADIAZINE 1,1-DIOXYDE

La solution de 0,8 g de 3-méthylthio-4H-pyrido[4,3-e] [1,2,4]thiadiazine 1,1-dioxyde obtenu à l'exemple 39 dans 8 cm³ de 1,2-diméthylpropylamine est portée à reflux pendant 2 jours. Après élimination du solvant sous dépression, le résidu est repris par 10 cm³ d'eau et le pH est ajusté à 7. La suspension aqueuse est extraite par trois fractions de CHCl₃. La phase organique est séchée (MgSO₄) et concentrée à siccité sous dépression. L'huile obtenue, reprise par un petit volume d'acétate d'éthyle et placée une nuit à + 4°C, laisse précipiter le composé du titre. Il est recueilli sur filtre, lavé à l'éther de pétrole (40-60°C) et séché.
Rdt : 80-85 %
P.F. : 199-202°C.

### EXEMPLE 46 : 3-CYCLOHEXYLAMINO-4H-PYRIDO[4,3-e][1,2,4]THIADIAZINE 1,1-DIOXYDE

La solution de 0,7 g de 3-méthylthio-4H-pyrido[4,3-e] [1,2,4]thiadiazine 1,1-dioxyde dans 7 cm³ de cyclohexylamine, est chauffée à reflux pendant 4 heures. Le milieu réactionnel est concentré sous dépression, et le résidu est repris par 10 cm³ d'eau et est basifiée légèrement par adjonction de soude à 10 %. La phase aqueuse est agitée avec 25 cm³ de diéthyléther afin d'extraire un excès éventuel de cyclohexylamine. Après décantation, la solution aqueuse est ajustée à pH 7 et le précipité blanc est recueilli sur filtre et lavé à l'eau. Il est recristallisé dans l'eau chaude.
Rdt : 90-95 %
P.F. (monohydrate) : 134-137°C.

### EXEMPLES 47 ET 48

En procédant comme dans l'exemple 46 mais en utilisant les amines appropriées, on obtient les composés des exemples suivants :

### EXEMPLE 47 : 3-CYCLOPENTYLAMINO-4H-PYRIDO[4,3-e][1,2,4]THIADIAZINE 1,1-DIOXYDE

Rd : 90-95 %
P.F (monohydrate) : 237-240°C.

### EXEMPLE 48 : 3-(4-MORPHOLINYL)AMINO-4H-PYRIDO[4,3-e][1,2,4]THIADIAZINE 1,1-DIOXYDE

Rdt : 70-75 %
P.F. (monohydrate) : 291-293°C.

### EXEMPLE 49 : 6-CHLORO-2,3-DIHYDRO-4H-PYRIDO[4,3-e][1,2,4]THIADIAZINE 1,1-DIOXYDE

Le composé du titre est obtenu à partir de (4-amino-2-chloropyrid-5-yl)sulfonamide (obtenu au stade A de la préparation 19) dans les conditions expérimentales décrites pour l'exemple 1.
Rdt : 65 %
P.F. : 202-206°C.

### EXEMPLE 50 : 3-AMINO-4H-PYRIDO[3,2-e][1,2,4]THIADIAZINE 1,1-DIOXYDE

Le mélange intime de 1 g de (3-aminopyrid-2-yl)sulfonamide (préparation 1) et de 2,1 g de carbonate de guanidine est amené progressivement à l'état fondu (200°C), puis chauffé durant 24 heures à cette température. Après refroidissement, le résidu est repris par de l'eau. L'insoluble éventuel est filtré, et le filtrat, traité au noir animal, est acidifié jusqu'à pH 7. Le précipité blanc est recueilli sur filtre, lavé à l'eau et recristallisé dans l'eau chaude.
Rdt : 35-40 %
P.F. (monohydrate) : > 330°C.

### EXEMPLE 51 : 3-ETHYL-2,3-DIHYDRO-4H-PYRIDO[4,3-e][1,2,4]THIADIAZINE 1,1-DIOXYDE

0,5 g de (4-aminopyrid-3-yl)sulfonamide (préparation 2) et 0,34 g (2 équivalents) de propionaldéhyde sont introduits dans 5 cm³ d'isopropanol additionné de 10 gouttes de solution saturée d'acide chlorhydrique dans l'acétate d'éthyle. Après 2h de reflux, la solution obtenue est concentrée à siccité et le résidu est recristallisé dans le mélange isopropanol/éther de pétrole (40-60°C) 1:3.
Rdt : 75-80 %
P.F. : 215-217°C.

### EXEMPLE 52 : 3-PROPYL-2,3-DIHYDRO-4H-PYRIDO[4,3-e][1,2,4]THIADIAZINE 1,1-DIOXYDE

En procédant comme dans l'exemple 51, mais en remplaçant le propionaldéhyde par le butyraldéhyde, on obtient le composé du titre.
Cependant, après 2h de reflux, la suspension de cristaux obtenue est refroidie. Les cristaux sont recueillis sur filtre, lavés à l'isopropanol, séchés et recristallisés dans le mélange méthanol/diéthyléther 1:2.
Rdt : 70-75 %
P.F. : 229-234°C.

### EXEMPLES 53 A 56 :

En procédant comme dans l'exemple 52 mais en remplaçant le butyraldéhyde par l'aldéhyde approprié, on obtient les composés des exemples suivants :

### EXEMPLE 53 : 3-ISOPROPYL-2,3-DIHYDRO-4H-PYRIDO[4,3-e][1,2,4]THIADIAZINE 1,1-DIOXYDE

Rdt : 75-80 %
P.F. : 250-254°C.

### EXEMPLE 54 : 3-ETHYLPROPYL-2,3-DIHYDRO-4H-PYRIDO[4,3-e][1,2,4]THIADIAZINE 1,1-DIOXYDE

Rdt : 75-80 %
P.F. : 224-226°C.

### EXEMPLE 55 : 3-CYCLOHEXYL-2,3-DIHYDRO-4H-PYRIDO[4,3-e][1,2,4]THIADIAZINE 1,1-DIOXYDE

Solvant de recristallisation : méthanol/eau 1:2.
Rdt : 75-80 %
P.F. : 272-276°C.

### EXEMPLE 56 : 3-PHENYL-2,3-DIHYDRO-4H-PYRIDO[4,3-e][1,2,4]THIADIAZINE 1,1-DIOXYDE

Solvant de recristallisation : isopropanol/éther de pétrole (40-60°C) 1:3.
Rdt : 70-75 %
P.F. : 214-216°C.

### EXEMPLE 57 : 3-(CHLOROMETHYL)-2,3-DIHYDRO-4H-PYRIDO[4,3-e] [1,2,4]THIADIAZINE 1,1-DIOXYDE

Rdt : 70-75%
P.F (chlorhydrate) : 264-267°C

### EXEMPLE 58 : 2-METHYL-2,3-DIHYDRO-4H-PYRIDO[4,3-e][1,2,4]THIADIAZINE 1,1-DIOXYDE

Le composé du titre est obtenu à partir de N-méthyl-(4-aminopyrid-3-yl)sulfonamide (préparation 12) en appliquant les conditions expérimentales décrites pour l'exemple 9. Cependant, après 24 heures de reflux, le solvant est éliminé sous dépression. Le résidu est purifié sur colonne de silice en utilisant le mélange chloroforme/méthanol 95:5 comme phase éluante. Le composé recueilli est ensuite recristallisé dans le mélange chloroforme/méthanol 4:1.
Rdt : 40-45 %
P.F. : 209-211°C.

### EXEMPLE 59 : 4-ETHYL-2,3-DIHYDRO-4H-PYRIDO[4,3-e][1,2,4]THIADIAZINE 1,1-DIOXYDE

Le composé de l'exemple 59 est obtenu à partir de (4-éthylaminopyrid-3-yl)sulfonamide (préparation 21) en appliquant les conditions expérimentales décrites dans l'exemple 3
Rdt : 90-95 %
P.F : 229-230°C

### EXEMPLE 60 : 4-PROPYL-2,3-DIHYDRO-4H-PYRIDO[4,3-e][1,2,4]THIADIAZINE 1,1-DIOXYDE

Le composé de l'exemple 60 est obtenu à partir de (4-propylaminopyrid-3-yl)sulfonamide (préparation 22) en appliquant les conditions expérimentales décrites dans l'exemple 3.
Rdt : 75-80 %
P.F : 160-163°C

### EXEMPLE 61 : 4-BUTYL-2,3-DIHYDRO-4H-PYRIDO[4,3-e][1,2,4]THIADIAZINE 1,1-DIOXYDE

Le composé est obtenu à partir de (4-butylaminopyrid-3-yl)sulfonamide en appliquant les conditions expérimentales décrites pour l'exemple 3. Le chlorhydrate correspondant est obtenu en dissolvant le composé base dans l'acétate d'éthyle et en ajoutant une solution saturée d'acide chlorhydrique dans l'acétate d'éthyle. Le chlorhydrate précipite. Il est recueilli sur filtre, lavé à l'acétate d'éthyle et séché.
Rdt : 65-70 %
P.F : 277-280°C (Chlorhydrate)

### EXEMPLE 62 : 4-(2',2',2'-TRIFLUOROETHYL)-2,3-DIHYDRO-4H-PYRIDO [4,3-e][1,2,4]THIADIAZINE 1,1-DIOXYDE

Le composé est obtenu à partir de [4-(2' ,2' ,2'-trifluoroéthylamino)pyrid-3-yl]sulfonamide (préparation 24) en appliquant les conditions expérimentales décrites dans l'exemple 3.
Rdt : 70-75 %
P.F : 200-201°C

### EXEMPLE 63 : 4-METHYL-4H-PYRIDO[3,2-e][1,2,4]THIADIAZINE 1,1-DIOXYDE

A une solution de 0,6 g de 4H-pyrido[3,2-e][1,2,4]thiadiazine 1,1-dioxyde (exemple 16) dans 15 cm³ d'acétonitrile est ajouté 1,2 g de carbonate de potassium et 1,37 g d'iodométhane. La suspension est chauffée à 50°C pendant 3 heures. L'acétonitrile est ensuite éliminé par évaporation sous dépression. Le résidu est repris par 20 cm³ d'eau. Le produit du titre peu soluble dans l'eau est recueilli sur filtre, lavé à l'eau et séché.
Rdt : 60-65 %
P.F : 227-228°C

### EXEMPLE 64 : 4-METHYL-2,3-DIHYDRO-4H-PYRIDO[3,2-e][1,2,4]THIADIAZINE 1,1-DIOXYDE

A la solution de 0,1 g de 4-méthyl-4H-pyrido[3,2-e][1,2,4]thiadiazine 1,1-dioxyde (exemple 63) dans 6 cm³ d'isopropanol est ajouté 0,08 g de NaBH₄. Le mélange est maintenu sous agitation à température ambiante pendant 45 minutes. Le solvant est ensuite éliminé sous dépression et le résidu est additionné de 5 cm³ d'eau. La suspension obtenue est ajustée à pH 7 et ensuite extraite par du chloroforme (3 fois 100 cm³). La phase organique est lavée à l'eau (25 cm³), séchée (MgSO₄), puis concentrée à siccité pour fournir le composé du titre.
Rdt : 95-100 %
P.F : 208-210°C

### EXEMPLE 65 : 4-ETHYL-4H-PYRIDO[3,2-e][1,2,4]THIADIAZINE 1,1-DIOXYDE

Le composé est obtenu selon la même procédure que celle décrite pour l'exemple 63 au départ de 0,1 g de 4H-pyrido[3,2-e][1,2,4]thiadiazine 1,1-dioxyde (exemple 16) et de 0,17 g de bromure d'éthyle.
Rdt : 65-70 %
P.F : 154-156°C

### EXEMPLE 66 : 4-ETHYL-2,3-DIHYDRO-4H-PYRIDO[3,2-e][1,2,4]THIADIAZINE 1,1-DIOXYDE

Le composé est obtenu selon la même procédure que celle décrite pour l'exemple 64 au départ de 4-ethyl-4H-pyrido[3,2-e][1,2,4]thiadiazine 1,1-dioxyde (exemple 65).
Rdt : 85-90%
P.F : 190-191°C

### EXEMPLE 67 : 3,4-DIMETHYL-2,3-DIHYDRO-4H-PYRIDO[4,3-e][1,2,4]THIADIAZINE 1,1-DIOXYDE

La solution de 0,5 g de (4-méthylaminopyrid-3-yl)sulfonamide (préparation 12) et de 0,75 cm³ d'acétaldéhyde dans 5 cm³ d'isopropanol, additionnée de 3 gouttes de solution saturée d'acide chlorhydrique dans l'acétate d'éthyle, est chauffée pendant 2 heures à 50°C. Le solvant est ensuite éliminé sous dépression et le résidu obtenu est recristallisé une première fois dans l'isopropanol, puis dans le méthanol.
Rdt : 45-50 %
P.F : 189-190°C

### EXEMPLE 68 : 3-ETHYL-4-METHYL-2,3-DIHYDRO-4H-PYRIDO[4,3-e] [1,2,4]THIADIAZINE 1,1-DIOXYDE

La solution de 0,5 g de (4-méthylaminopyrid-3-yl)sulfonamide (préparation 12) et de 0,9 cm³ de propionaldéhyde dans 5 cm³ d'isopropanol, additionnée de 3 gouttes de solution saturée d'acide chlorhydrique dans l'acétate d'éthyle, est chauffée pendant 3 heures à 50°C. Le solvant est ensuite éliminé sous dépression et le résidu obtenu est recristallisé plusieurs fois dans le mélange chloroforme-éther de pétrole (1:3).
Rdt : 35-40 %
P.F : 147-149°C

### EXEMPLE 69 : 2-METHYL-2,3-DIHYDRO-4H-PYRIDO[3,2-e] [1,2,4]THIADIAZINE 1,1-DIOXYDE

A la solution de 0,25 g de 2,3-dihydro-4H-pyrido[3,2-e] [1,2,4]thiadiazine 1,1-dioxyde (exemple 1) dans 10 cm³ d'acétonitrile sont ajoutés 0,52 g de carbonate de potassium et 0,94 g d'iodométhane. La suspension est chauffée à 50°C pendant 3 heures. L'acétonitrile est ensuite éliminé par évaporation sous dépression et le résidu obtenu est repris par 10 cm³ d'eau. La suspension est extraite par le chloroforme (5 fois 100 cm³). Le solvant d'extraction est séché sur MgSO₄, puis concentré à siccité. Le solide obtenu est purifié par chromatographie sur colonne de silice (solvant d'élution MeOH/CHCl₃ : 1/9).
Rdt : 60-65 %
P.F : 182-183°C

### EXEMPLE 70 : 2,4-DIMETHYL-2,3-DIHYDRO-4H-PYRIDO[4,3-e][1,2,4]THIADIAZINE 1,1-DIOXYDE

La solution de 0,25 g de 4-méthyl-2,3-dihydro-4H-pyrido[4,3-e] [1,2,4]thiadiazine 1,1-dioxyde (exemple 5) dans 8 cm³ d'acétonitrile est additionnée de 0,5 g de carbonate de potassium (3éq.), puis de 0,26 g de p-toluènesulfonate de méthyle (1,1 éq.).La suspension obtenue est portée à reflux pendant 2 heures. Ensuite, le solvant est éliminé par évaporation sous pression réduite et le résidu est partagé entre 10 cm³ d'eau et 100 ml de chloroforme. La phase aqueuse est encore extraite deux fois par 50 cm³ de chloroforme. Les fractions organiques rassemblées sont séchées sur MgSO₄, filtrées, puis concentrées à siccité. Le résidu est dissous dans un minimum de chloroforme. L'addition d'un excès d'éther de pétrole (40-60°C) fait précipiter des cristaux de produit du titre. Ils sont recueillis sur filtre, lavés à l'éther de pétrole (40-60°C) et séchés.
Rdt : 60-65 %
P.F : 167-169°C

### EXEMPLE 71 : 4-METHYL-2-PROPYL-2,3-DIHYDRO-4H-PYRIDO[4,3-e] [1,2,4]THIADIAZINE 1,1-DIOXYDE

Le composé du titre est obtenu selon la procédure décrite pour l'exemple 70 au départ de 4-méthyl-2,3-dihydro-4H-pyrido[4,3-e] [1,2,4]thiadiazine 1,1-dioxyde (exemple 5), mais en utilisant l'hydrure de sodium (2 éq.) et le bromure de propane (3 éq. ) au lieu de carbonate de potassium et de p-toluènesulfonate de méthyle, respectivement.
Rdt : 50-55 %
P.F : 130-131°C

### EXEMPLE 72 : 4-ISOPROPYL-2-METHYL-2,3-DIHYDRO-4H-PYRIDO[4,3-e] [1,2,4]THIADIAZINE 1,1-DIOXYDE

Le composé du titre est obtenu selon la procédure décrite pour l'exemple 71 au départ de 4-isopropyl-2,3-dihydro-4H-pyrido[4,3-e] [1,2,4]thiadiazine 1,1-dioxyde (exemple 3), mais en utilisant le p-toluènesulfonate de méthyle (1,5 éq.) au lieu du bromure de propane, et en limitant la durée du reflux à 1 heure.
Rdt : 45-50 %
P.F : 159-161°C

### EXEMPLE 73 : 2,4-DIMETHYL-2,3-DIHYDRO-4H-PYRIDO[3,2-e][1,2,4]THIADIAZINE 1,1-DIOXYDE

Le composé du titre est obtenu selon la même procédure que celle décrite pour l'exemple 69 au départ de 0,3 g de 4-méthyl-2,3-dihydro-4H-pyrido [3,2-e][1,2,4]thiadiazine 1,1-dioxyde (exemple 64), de 0,62 g de carbonate de potassium et de 0,63 g d'iodométhane en limitant la durée du chauffage à 50°C à 1 heure et demi. Le produit obtenu est transformé en chlorhydrate après dissolution dans 10 cm³ d'acétate d'éthyle et addition de 4 cm³ d'acétate d'éthyle saturé d'acide chlorhydrique gazeux. Le précipité obtenu est recueilli sur filtre, lavé à l'acétate d'éthyle et séché.
Rdt : 50-55 %
P.F (chlorhydrate) : 172-173°C

### EXEMPLE 74 : 4-ETHYL-2-METHYL-2,3-DIHYDRO-4H-PYRIDO[3,2-e] [1,2,4]THIADIAZINE 1,1-DIOXYDE

Le composé du titre est obtenu selon la même procédure que celle décrite pour l'exemple 70 au départ de 4-éthyl-2,3-dihydro-4H-pyrido[3,2-e] [1,2,4]thiadiazine 1,1-dioxyde (exemple 66).
Rdt : 60-65 %
P.F : 103-104°C

### EXEMPLE 75 : 6-METHYL-4H-PYRIDO[3,2-e][1,2,4]THIADIAZINE 1,1-DIOXYDE

La suspension de 1,3 g de (3-amino-5-méthylpyrid-2-yl)sulfonamide (préparation 25) dans 13 cm³ d'orthoformiate d'éthyle est portée à reflux pendant 2 heures. Après refroidissement, le précipité obtenu est recueilli sur filtre, lavé à l'éther diéthylique et séché.
Rdt : 95-100%
P.F : 322-324°C

### EXEMPLE 76 : 4,6-DIMETHYL-4H-PYRIDO[3,2-e][1,2,4]THIADIAZINE 1,1-DIOXYDE

Une solution de 0,6 g de 6-méthyl-4H-pyrido[3,2-e][1,2,4]thiadiazine 1,1-dioxyde (exemple 75) dans 15 cm³ d'acétonitrile est additionnée de 2,5 g de carbonate potassique et de 0,9 g de p-toluènesulfonate de méthyle. Après 16 heures de reflux, le solvant est éliminé sous dépression, et le solide obtenu est repris par 15 cm³ d'eau. Le précipité est recueilli sur filtre, lavé à l'eau et séché.
Rdt :75-80%
P.F : 247-249°C

### EXEMPLE 77 : 4,6-DIMETHYL-2,3-DIHYDRO-4H-PYRIDO[3,2-e] [1,2,4]THIADIAZINE 1,1-DIOXYDE

La solution de 0,5 g de 4,6-diméthyl-4H-pyrido[3,2-e][1,2,4]thiadiazine 1,1-dioxyde (exemple 76) dans 25 cm³ du mélange isopropanol/chloroforme 1:1 est additionnée par petites fractions de 0,4 g de borohydrure de sodium sous bonne agitation et sous atmosphère inerte. Après 30 minutes à température ambiante, l'excès de borohydrure est détruit par addition de quelques gouttes d'acide acétique, et le solvant est éliminé sous pression réduite. Le résidu est repris par 10 cm³ d'eau et le pH du milieu est amené à la neutralité. Le précipité obtenu est recueilli sur filtre, lavé à l'eau et séché.
Rdt : 75-80%
P.F : 220-221°C

### EXEMPLE 78 : 4-ETHYL-6-METHYL-4H-PYRIDO[3,2-e][1,2,4]THIADIAZINE 1,1-DIOXYDE

Une solution de 1,2 g de 6-méthyl-4H-pyrido[3,2-e][1,2,4]thiadiazine 1,1-dioxyde (exemple 75) dans 15 cm³ d'acétonitrile est additionnée de 2,5 g de carbonate potassique et de 2 g de bromure d'éthyle. Après 16 heures de reflux, le solvant est éliminé sous dépression, et le solide obtenu est repris par 15 cm³ d'eau. Le précipité est recueilli sur filtre, lavé à l'eau et séché.
Rdt : 60-65%
P.F : 185-187°C

### EXEMPLE 79 : 4-ETHYL-6-METHYL-2,3-DIHYDRO-4H-PYRIDO[3,2-e] [1,2,4]THIADIAZINE 1,1-DIOXYDE

La solution de 0,6 g de 4-éthyl-6-méthyl-4H-pyrido[3,2-e] [1,2,4]thiadiazine 1,1-dioxyde (exemple 78) dans 30 cm³ du mélange isopropanol/chloroforme 1:1 est additionnée par petites fractions de 0,45 g de borohydrure de sodium sous bonne agitation et sous atmosphère inerte. Après 30 minutes à température ambiante, l'excès de borohydrure est détruit par addition de quelques gouttes d'acide acétique, et le solvant est éliminé sous pression réduite. Le résidu est repris par 10 cm³ d'eau et le pH du milieu est amené à la neutralité. Le précipité obtenu est recueilli sur filtre, lavé à l'eau et séché.
Rdt : 60-65 %
P.F : 146-148°C

### EXEMPLE 80 : 2,6-DIMETHYL-4-ETHYL-2,3-DIHYDRO-4H-PYRIDO[3,2-e] [1,2,4]THIADIAZINE 1,1-DIOXYDE

La solution de 0,2 g de 4-éthyl-6-méthyl-2,3-dihydro-4H-pyrido[3,2-e] [1,2,4]thiadiazine 1,1-dioxyde (exemple 79) dans 10 cm³ d'acétonitrile est additionnée de 2 équivalents de NaOH, puis portée à reflux pendant 15 minutes sous agitation. Le milieu est additionné de 0,18 g de p-toluènesulfonate de méthyle et agité pendant 2 heures à température ambiante. Le solvant est ensuite éliminé sous pression réduite et le résidu est repris par 5 cm³ d'eau. le précipité est recueilli sur filtre, lavé à l'eau et séché. Il est purifié par chromatographie sur colonne de silice (chloroforme-éther diéthylique 1:1).
Rdt : 50-55%
P.F : 121-122°C

### EXEMPLE 81 à 83 : 4-ETHYL-2-ALKYL(ARYL)OXYCARBONYL-2,3-DIHYDRO-4H-PYRIDO[3,2-e][1,2,4]THIADIAZINE 1,1-DIOXYDE

### Mode opératoire général :

Dans un ballon de 50 cm³ sont introduites 9,4 x 10⁻⁴ moles de 4-éthyl-2,3-dihydro-4H-pyrido[3,2-e][1,2,4]thiadiazine 1,1-dioxyde (exemple 66), 3,8 x 10⁻³ moles (4 équivalents) de carbonate potassique et 17 cm³ d'acétonitrile. A cette suspension sont ajoutées sous agitation 1,3 x 10⁻³ moles (1,2 équivalents) de chloroformiate d'alkyle (ou d'aryle). Après 4 heures à température ambiante, l'insoluble (carbonate potassique) est éliminé par filtration, et le filtrat est concentré à siccité sous pression réduite. Le résidu est repris par 10 cm³ d'éther de pétrole (40-60°C), séché, lavé à l'eau, et séché à nouveau. Les composés ainsi obtenus sont purs.

### EXEMPLE 81 : 4-ETHYL-2-METHYLOXYCARBONYL-2,3-DIHYDRO-4H-PYRIDO[3,2-e] [1,2,4]THIADIAZINE 1,1-DIOXYDE

Rdt : 70-75%
P.F : 138-140°C

### EXEMPLE 82 : 4-ETHYL-2-ETHYLOXYCARBONYL-2,3-DIHYDRO-4H-PYRIDO[3,2-e] [1,2,4]THIADIAZINE 1,1-DIOXYDE

Rdt : 60-65%
P.F : 50-55°C (décomposition)

### EXEMPLE 83 : 4-ETHYL-2-PHENYLOXYCARBONYL-2,3-DIHYDRO-4H-PYRIDO[3,2-e] [1,2,4]THIADIAZINE 1,1-DIOXYDE

Rdt : 50-55%
P.F : 90-95°C(décomposition)

### EXEMPLES PHARMACOLOGIQUES

### EXEMPLE A : Etude des courants excitateurs induit par l'AMPA dans les ovocytes de Xenopus

### a) Méthode :

Des ARNm sont préparés à partir de cortex cérébral de rat mâle Wistar par la méthode au guanidinium thiocyanate/phénol/chloroforme. Les ARNm Poly (A+) sont isolés par chromatographie sur oligo-dT cellulose et injectés à raison de 50 ng par ovocyte. Les ovocytes sont laissés 2 à 3 jours en incubation à 18°C pour permettre l'expression des récepteurs puis stockés à 8-10°C.

L'enregistrement électrophysiologique est réalisé dans une chambre en plexiglass® à 20-24°C en milieu OR2 (J. Exp. Zool., 1973, 184, 321-334) par la méthode du "voltage-clamp" à 2 électrodes, une 3ème électrode placée dans le bain servant de référence.

Tous les composés sont appliqués via le milieu d'incubation et le courant électrique est mesuré à la fin de la période d'application. L'AMPA est utilisé à la concentration de 30 µM. Pour chaque composé étudié, on définit la concentration doublant (EC2X) ou quintuplant (EC5X) l'intensité du courant induit par l'AMPA seul (50 à 100 nA).

### b) Résultats :

Les composés de l'invention potentialisent très fortement les effets excitateurs de l'AMPA et leur activité est très nettement supérieure aux composés de référence comme le montre le tableau ci-dessous :

| | **EC2X (**µ**M)** | **EC5X (**µ**M)** |
|---|---|---|
| **Diazoxide** | 400 | 1 000 |
| **Aniracetam** | 1 300 | 5 000 |
| **Exemple 12** | 200 | 400 |
| **Exemple 25** | 250 | 800 |
| **Exemple 5** | 50 | 300 |
| **Exemple 3** | 90 | 250 |
| **Exemple 8** | 200 | 500 |
| **Exemple 14** | 300 | 400 |
| **Exemple 49** | 500 | 800 |
| **Exemple 64** | 29 | 37 |
| **Exemple 66** | 10 | 16 |

### EXEMPLE B : Etude des potentiels excitateurs synaptiques induits par la stimulation électrique sur coupe d'hippocampe

### a) Méthode :

Des coupes transversales d'hippocampe (500 µM) de rat mâle Wistar sont préparées à l'aide d'un "tissu chopper" puis incubées durant 45 minutes dans un milieu sans calcium contenant 10 mM Mg⁺⁺. Elles sont ensuite stabilisées dans du Krebs ajusté à pH 7,35 et oxygéné par O₂/CO₂ (95 % / 5 %) à température ambiante.

Les coupes sont mises en submersion à 35°C et les potentiels excitateurs post-synaptiques (PEPS) sont enregistrés dans le champ dendritique des cellules granulaires du dentate gyrus lors de stimulation (50-100 µA, 50 µsec) toutes les 30 secondes de la voie perforante par électrode bipolaire de tungstène.

L'acquisition et l'analyse des PEPS sont réalisées grâce à un convertisseur A-D, une interface TL-1 et un software "pCLAMP".

L'amplitude et la durée des PEPS sont évaluées sur l'onde négative par rapport au courant de base.

Les composés sont appliqués durant 10 à 20 minutes dans le bain de superfusion contenant du MgSO₄ (1 mM) afin de bloquer l'activation des récepteurs NMDA. Pour chaque composé, on définit la concentration augmentant de 50 % l'amplitude (A50) ou la durée (D50) du PEPS.

### b) Résultats :

Les composés de l'invention augmentent la durée du PEPS à doses plus faibles que les composés de référence comme le montre le tableau suivant :

| | **D50(**µ**M)** |
|---|---|
| **Aniracetam** | 3 000 |
| **Diazoxide** | 560 |
| **Exemple 12** | 100 |
| **Exemple 5** | 130 |
| **Exemple 3** | 240 |
| **Exemple 49** | 300 |

De plus, les composés de l'invention exercent des effets originaux par rapport aux composés de référence et notamment au Diazoxide. En effet, ce dernier exerce davantage ses effets excitateurs vis-à-vis de la durée du PEPS comme le montre le rapport D50/A50 qui est égal à 0,5. Par contre, les dérivés de l'invention peuvent soit augmenter préférentiellement la durée, soit préférentiellement l'amplitude comme le montre le tableau suivant :

| | **D50/A50** |
|---|---|
| **Diazoxide** | 0,5 |
| **Exemple 5** | 0,6 |
| **Exemple 12** | 0,65 |
| **Exemple 3** | 0,9 |
| **Exemple 14** | 1,25 |
| **Exemple 49** | 1,55 |

### EXEMPLE C : Etude des effets facilitateurs de l'excitation cérébrale induite par un stress auditif chez la Souris DBA2

### a) Méthode :

Des souris DBA2 (Iffa-Credo, l'Arbresic, France) âgées de 21 à 26 jours sont soumises à un stress sonore dans une enceinte isolée.

Ce stress entraîne des symptômes d'excitation puis des convulsions s'il est appliqué à forte intensité (1.400 Hz, 100 dB). Ces conséquences comportementales sont antagonisées par les composés bloquant la neurotransmission glutamatergique.

Appliqué à faible fréquence (1.800 Hz, 100 dB), le stress sonore entraîne peu ou pas de symptômes d'excitation.

Les composés facilitant la neurotransmission glutamatergique peuvent potentialiser ces symptômes qui sont alors cotés pour chaque animal de 1 à 4 selon leur intensité.

Chaque groupe d'animaux (n=10) reçoit le composé par voie IP, 30 minutes avant le stress sonore. Un groupe contrôle (n=10) reçoit le solvant.

La dose doublant le score d'excitation par rapport au score témoin est mesurée.

### b) Résultat :

La potentialisation in vitro des courants induits par l'AMPA s'exprime in vivo puisque le composé de l'exemple 66 dont l'EC2X est de 10 µM, double le score d'excitation à la dose de 1 mg/Kg IP. Dans les mêmes conditions d'administration, le diazoxide et l'aniracetam sont inactifs.

### EXEMPLE D : Recherche d'effets toxiques

Administrés par voie orale, les composés de l'invention n'entraînent ni mortalité, ni symptômes comportementaux signant une toxicité, jusqu'à la dose maximale testée de 300 mg/kg.

## Revendications

1. composés de formule (I) : 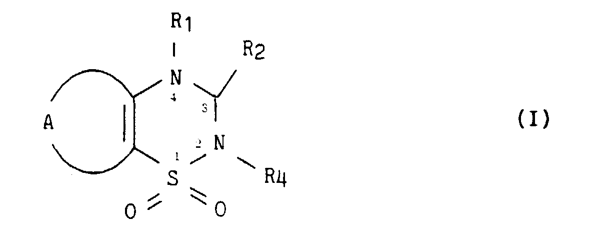 dans laquelle :
- R₁ représente un groupement choisi parmi :
. hydrogène,
. R₃ avec R₃ représentant un radical choisi parmi alkyle inférieur, alcényle inférieur et alcynyle inférieur, R₃ étant non substitué ou substitué,
. cycloalkyle,
. cycloalkyle substitué,
. cycloalkylalkyle inférieur,
. cycloalkylalkyle inférieur substitué,
. acyle inférieur,
. alkoxycarbonyl inférieur,
. arylcarbonyl non substitué ou substitué sur le noyau aryle,
. aryloxycarbonyl non substitué ou substitué sur le noyau aryle,
ou R₁ forme entre les atomes 3 et 4 du cycle thiadiazinique, présent dans la formule (I), une double liaison ;
- R₂ représente un groupement choisi parmi :
. hydrogène,
. R₁₀ avec R₁₀ ayant la même définition que R₃ ci-dessus, R₁₀ étant non substitué ou substitué,
. R₁₁ avec R₁₁ représentant un groupement choisi parmi cycloalkyle, cycloalkylalkyle inférieur, bicycloalkyle, et bicycloalkylalkyle inférieur, R₁₁ étant non substitué ou substitué,
. O-R₁₀ avec R₁₀ tel que défini précédemment,
. thioxo,
. -S-R₁₂, dans lequel R₁₂ représente un alkyle inférieur, R₁₂ étant non substitué ou substitué,
. aryle,
. aryle substitué,
. et 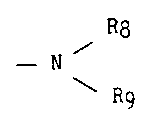 dans lequel R₈ et R₉ représentent, indépendamment l'un de l'autre, un radical choisi parmi **:**
. hydrogène,
. alkyle inférieur,
. cycloalkyle,
. cycloalkylalkyle inférieur,
. un hétérocycle R₁₃ choisi parmi pyrrolidine, pipéridine, pipérazine, morpholine, et thiomorpholine ;
. ou forment ensemble avec l'atome d'azote qui les porte un hétérocycle R₁₃ tel que défini précédemment,
- R₄ représente un groupement choisi parmi :
. hydrogène,
. R₁₄, R₁₄ ayant la même définition que R₃ ci-dessus, R₁₄ étant non substitué ou substitué,
. cycloalkyle,
. aryle,
. aryle substitué,
. acyle inférieur,
. alkoxycarbonyl inférieur,
. arylcarbonyl non substitué ou substitué sur le noyau aryle,
. aryloxycarbonyl non substitué ou substitué sur le noyau aryle,
ou
R₄ forme entre les atomes 2 et 3 du cycle thiadiazinique, présent dans la formule (I), une double liaison ;
- A forme avec les 2 atomes de carbone qui le portent un noyau pyridinique choisi parmi les groupements A₁, A₂, A₃ et A₄ : 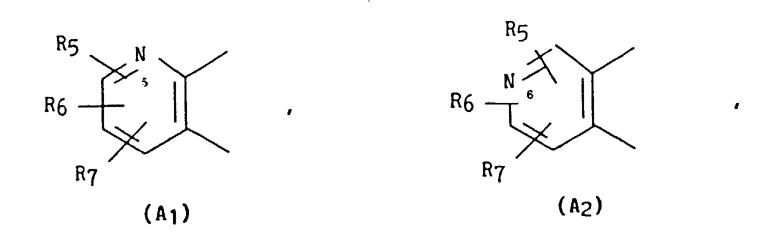 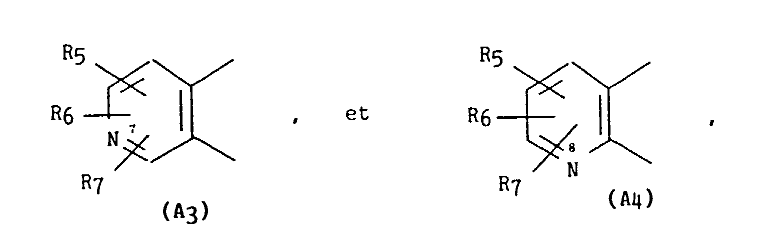 dans lesquels R₅, R₆ et R₇ représentent, indépendamment l'un de l'autre, un radical choisi parmi : hydrogène, halogène, alkyle inférieur, hydroxy, thiol, alkoxy inférieur, alkyle-thio inférieur, trifluorométhyle, carboxyle, acyle inférieur, aryle, arylalkyle inférieur, amino, alkylamino inférieur et dialkylamino inférieur,
étant entendu que lorsque A représente un groupement de formule A₁ non substitué, R₂ représente un atome d'hydrogène, un radical amino ou un radical méthyle, et R₅, R₆, R₇ représentent simultanément des atomes d'hydrogène, alors R₁ ne peut pas être un atome d'hydrogène ou un radical méthyle,
étant entendu que le composé de formule (I) ne peut représenter le 4H-pyrido[4,3-e]-1,2,4-thiadiazine-1,1-dioxyde,
étant entendu que, sauf précisions contraires,
. "alkyle inférieur", "alkoxy inférieur", "acyle inférieur" et "alkylthio inférieur" signifient des groupements linéaires ou ramifiés de 1 à 6 atomes de carbone,
. "alcényl inférieur" signifie un groupement linéaire ou ramifié de 2 à 6 atomes de carbone comprenant de 1 à 2 double liaisons,
. "alcynyl inférieur" signifie un groupement linéaire ou ramifié de 2 à 6 atomes de carbone comprenant de 1 à 2 triple liaisons,
. "cycloalkyle" signifie un groupement cyclique de 3 à 8 atomes de carbone,
. "bicycloalkyle" signifie un groupement bicyclique de 6 à 12 atomes de carbone,
. "aryle" signifie phényle ou naphtyle,
. "substitué" associé aux groupements R₃, R₁₀, et R₁₄, signifie que ces groupements ainsi qualifiés sont substitués par un ou plusieurs radicaux choisis parmi halogène, hydroxy, et alkoxy inférieur,
. "substitué" associé aux groupements "cycloalkyle", "cycloalkylalkyle", et R₁₁, signifie que ce groupement ainsi qualifié est substitué par un ou plusieurs radicaux ou groupes choisis parmi oxo, hydroxy, alkyle inférieur, et alkoxy inférieur,
. "substitué" associé au groupement aryle signifie que ce groupement ainsi qualifié est substitué par un ou plusieurs radicaux choisis parmi halogène, hydroxy, alkyle inférieur, alkoxy inférieur, et trifluorométhyle,
- leurs isomères optiques,
- et leurs sels d'addition à un acide ou à une base pharmaceutiquement acceptable.

2. Composé selon la revendication 1 qui est le 4-isopropyl-2,3-dihydro-4H-pyrido[4,3-e][1,2,4]thiadiazine 1,1-dioxyde.

3. Composé selon la revendication 1 qui est le 3,4-diméthyl-4H-pyrido[4,3-e][1,2,4]thiadiazine 1,1-dioxyde.

4. Composé selon la revendication 1 qui est le 4-méthyl-2,3-dihydro-4H-pyrido[4,3-e][1,2,4]thiadiazine 1,1-dioxyde.

5. Composé selon la revendication 1 qui est le 2,3-diméthyl-2,3-dihydro-4H-pyrido[4,3-e][1,2,4]thiadiazine 1,1-dioxyde.

6. Composé selon la revendication 1 qui est le 4-méthyl-2,3-dihydro-4H-pyrido[3,2-e][1,2,4]thiadiazine 1,1-dioxyde.

7. Composé selon la revendication 1 qui est le 4-éthyl-2,3-dihydro-4H-pyrido[3,2-e][1,2,4]thiadiazine 1,1-dioxyde.

8. Procédé de préparation des composés de formule (I) selon la revendication 1 caractérisé en ce que l'on fait réagir un composé de formule (II) : 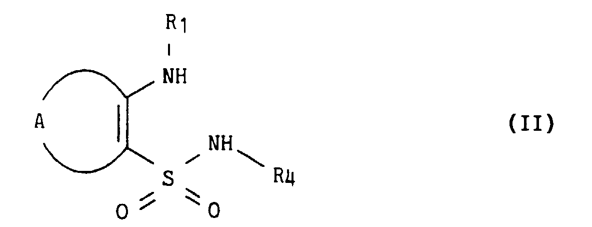 dans laquelle R₁, R₄ et A sont tels que définis dans la revendication 1,
a) - soit avec un composé de formule (III) 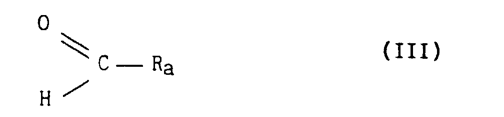 dans laquelle Ra représente un groupement R₁₀, R₁₁, aryle, ou aryle substitué, tels que définis dans la revendication 1,
ou, à chaud, avec un composé de formule (IV) : 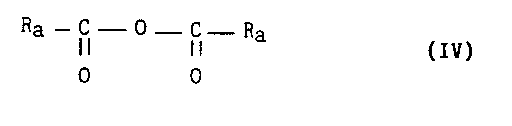 pour obtenir les composés de formule (I/a) : 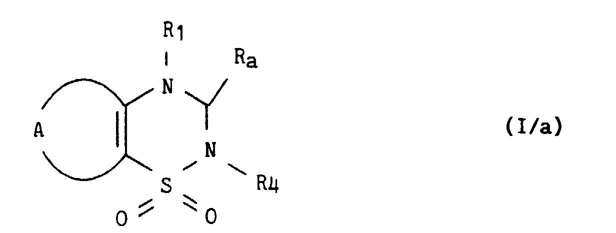 dans laquelle R₁, R₄, Rₐ et A sont tels que définis précédemment,
cas particulier des composés de formule (I) dans lesquels R₂ représente un groupement Rₐ,
- soit avec le paraformaldéhyde, l'orthoformiate d'éthyle, ou un anhydride mixte acide formique - acide acétique, pour obtenir les composé de formule (I/b) : 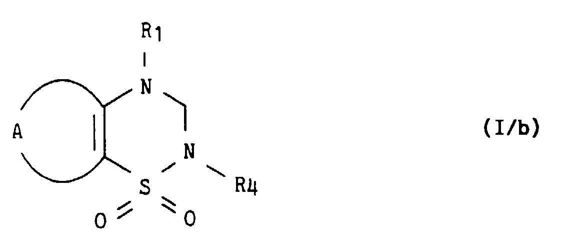 dans laquelle R₁, R₄ et A sont tels que définis précédemment,
cas particulier des composés de formule (I) dans lesquels R₂ représente un atome d'hydrogène,
ou bien,
b) - avec de l'urée pour conduire aux composés de formule (V) : 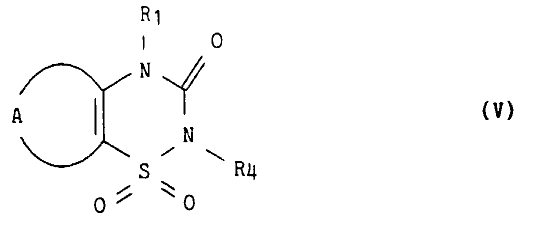 dans laquelle R₁, R₄, et A sont tels que décrits précédemment, qui sont ensuite :
* salifiés puis mis en réaction avec un composé de formule (VI) :
R₁₀―Hal (VI)
dans laquelle R₁₀ est tel que défini dans la revendication 1 et Hal représente un atome d'halogène,
pour obtenir un composé de formule (I/c) : 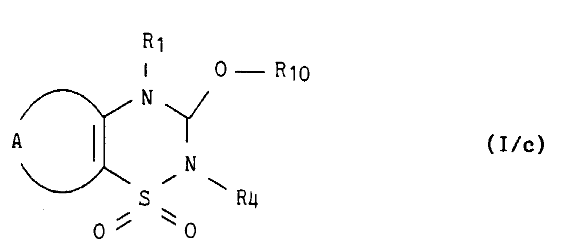 dans laquelle R₁, R₄, R₁₀ et A sont tels que définis précédemment, cas particulier des composés de formule (I) dans lesquels R₂ représente un groupement -O-R₁₀,
* ou bien soumis à un agent de thionation pour obtenir le composé de formule (I/d) : 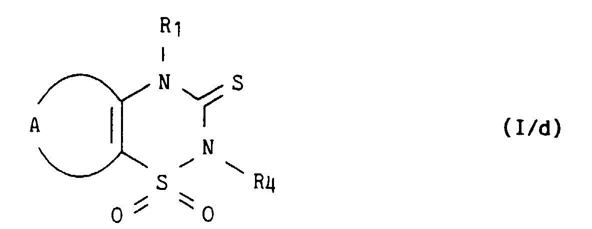 dans laquelle R₁, R₄, et A sont tels que définis précédemment, cas particulier des composés de formule (I) dans lesquels R₂ représente un groupement thioxo,
composé de formule (I/d) qui est mis en réaction avec un composé de formule (VI) :
Hal'―R₁₂ (VI)
dans laquelle R₁₂ est tel que défini dans la revendication 1 et Hal' représente un atome d'halogène pour obtenir le composé de formule (I/e) : 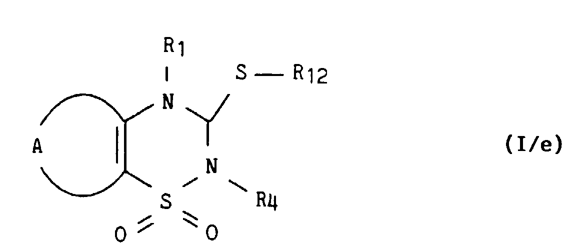 dans laquelle R₁, R₄, R₁₂, et A sont tels que définis précédemment, cas particulier des composés de formule (I) dans lesquels R₂ représente un groupement alkylthio,
composés de formule (I/e) qui peuvent être ensuite soumis à une amine de formule (VII) : 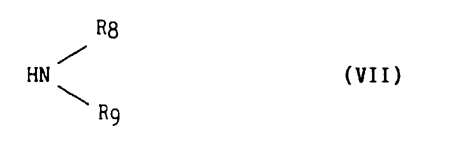 dans laquelle R₈ et R₉ sont tels que définis dans la revendication 1 pour obtenir les composés de formule (I/f) : 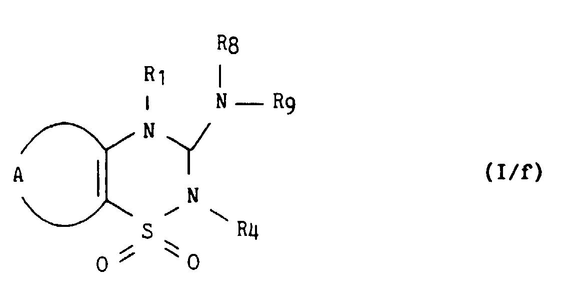 dans laquelle R₁, R₄, R₈, R₉, et A sont tels que définis précédemment, cas particulier des composés de formule (I) dans lesquels R₂ représente un groupement -NR₈R₉,
les composés de formule (I/a) à (I/f) formant l'ensemble des composés de formule (I),
étant entendu que les composés de formule (I/a), (I/b), (I/c), (I/e) et (I/f) peuvent être, le cas échéant, réduits sélectivement afin d'obtenir
les composés de formule (I) correspondants où la liaison entre les atomes 2 et 3, ou entre les atomes 3 et 4, du cycle thiadiazinique est saturée,
les composés de formule (I) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et salifiés par un acide ou une base pharmaceutiquement acceptable.

9. Procédé d'obtention des composés de formule (I/a), cas particulier des composés de formule (I) selon la revendication 1 : 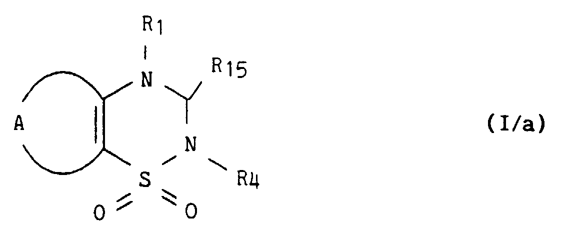 dans laquelle R₁, R₄, et A sont tels que définis dans la revendication 1, et R₁₅ représente un groupement R₁₀ ou R₁₁ tels que définis dans la revendication 1,
caractérisé en ce que :
on soumet un composé de formule (II/a) : 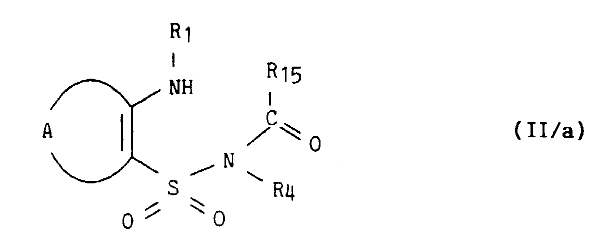 dans laquelle R₁, R₄, R₁₅ et A sont tels que définis précédemment à un agent cyclisant,
afin d'obtenir le composé de formule (I/a) correspondant,
les composés de formule (I/a) ainsi obtenus pouvant être :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et salifiés par un acide ou une base pharmaceutiquement acceptable.

10. Procédé de préparation des composés de formule (I/g), cas particulier des composés de formule (I) selon la revendiaction 1 : 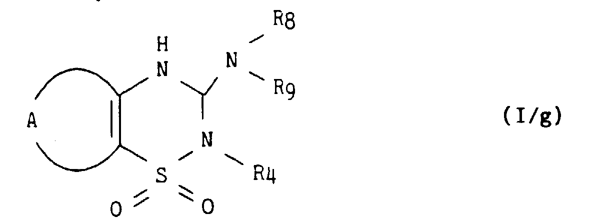 dans laquelle R₄, R₈, R₉, et A sont tels que définis dans la revendication 1, cas particulier des composés de formule (I) dans lesquels R₂ représente un groupement -NR₈R₉ et R₁ représente un atome d'hydrogène,
caractérisé en ce que :
- soit on fait réagir à chaud, en milieu basique, un composé de formule (II/b) : 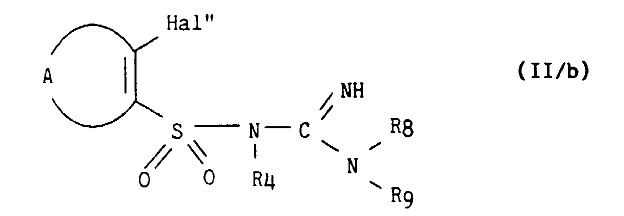 dans laquelle R₄, R₈, R₉, et A sont tels que définis précédemment, et Hal" représente un atome d'halogène,
- soit on fait réagir un composé de formule (II) : 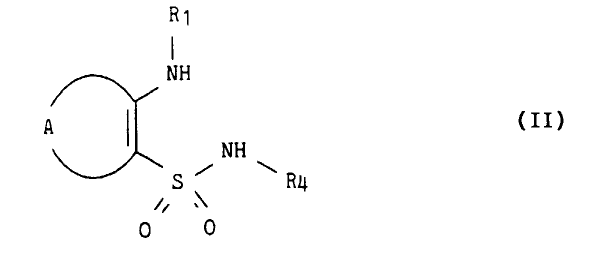 dans laquelle R₁, R₄, et A sont tels que définis précédemment, avec un composé de formule (VIII) : 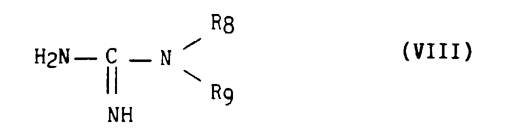 dans laquelle R₈ et R₉ sont tels que définis précédemment,
afin d'obtenir le composé de formule (I/g) correspondant,
les composés de formule (I/g) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et salifiés par un acide ou une base pharmaceutiquement acceptable.

11. Procédé de préparation des composés de formule (I/h), cas particulier des composés de formule (I) selon la revendication 1 : 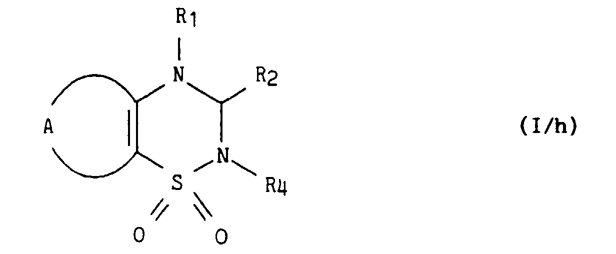 dans laquelle R₁, R₄, et A ont la même signification que dans la revendication 1, étant entendu qu'au moins un des substituants R₁ ou R₄ est différent d'un atome d'hydrogène,
caractérisé en ce que,
on fait réagir un composé de formula (I/i) : 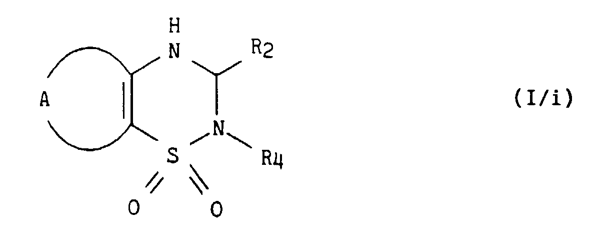 dans laquelle R₂, R₄, et A sont tels que définis précédemment avec un composé de formule (XI) :
R₁'―X (XI)
dans laquelle R₁' a la même signification que R₁ tel que défini dans la revendication 1 à l'exception de l'hydrogène et X représente un groupement partant,
et/ou, lorsque R₄ représente un atome d'hydrogène, avec un composé de formule (XII) :
R₄'―X' (XII)
dans laquelle R₄' a la même signification que R₄ tel que défini dans la revendication 1 à l'exception de l'hydrogène et X' représente un groupement partant,
les composés de formule (I/h) ainsi obtenus pouvant être :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange en leurs éventuels isomères optiques,
- et salifiés par un acide ou une base pharmaceutiquement acceptable.

12. Composition pharmaceutique contenant un composé selon la revendication 1 ou un de ses sels à un acide ou à une base pharmaceutiquement acceptable, en combinaison avec un ou plusieurs excipients ou véhicules pharmaceutiquement acceptables.

13. Composition pharmaceutique selon la revendication 12 contenant un composé selon la revendication 1 ou un de ses sels à un acide ou à une base pharmaceutiquement acceptable utile pour le traitement et la prévention des pathologies liées au dysfonctionnement de la neurotransmission glutamatergique.

14. Composition pharmaceutique selon la revendication 13 utile pour le traitement et la prévention des désordres mnémocognitifs associés à l'âge et aux syndromes anxieux ou dépressifs, des maladies neurodégénératives progressives, de la maladie d'Alzheimer, de la maladie de Pick, de la chorée d'Huntington, de la schizophrénie, des séquelles des maladies neurodégénératives aigües, des séquelles de l'ischémie et des séquelles de l'épilepsie.

## Patentansprüche

1. Verbindungen der Formel (I): 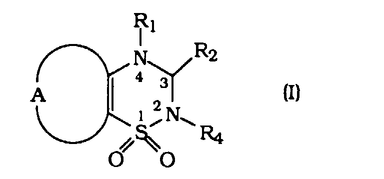 in der:
- R₁ eine Gruppe ausgewählt aus:
. Wasserstoff,
. R₃, worin R₃ eine Gruppe darstellt ausgewählt aus Niedrigalkyl, Niedrigalkenyl und Niedrigalkinyl und R₃ nichtsubstituiert oder substituiert ist,
. Cycloalkyl.
. substituiertes Cycloalkyl,
. Cycloalkylniedrigalkyl,
. substituiertes Cycloalkylniedrigalkyl,
. Niedrigacyl,
. Niedrigalkoxycarbonyl,
. nichtsubstituiertes oder am Arylkern substituiertes Arylcarbonyl,
. nichtsubstituiertes oder am Arylkern substituiertes Aryloxycarbonyl, bedeutet, oder R₁ zwischen den Atomen 3 und 4 des in der Formel (I) vorhandenen Thiadiazinrings eine Doppelbindung bildet;
- R₂ eine Gruppe ausgewählt aus:
. Wasserstoff,
. R₁₀, worin R₁₀ die gleichen Bedeutungen besitzt, wie sie oben für R₃ angegeben worden sind. wobei R₁₀ nichtsubstituiert oder substituiert ist,
. R₁₁, worin R₁₁ eine Gruppe ausgewählt aus Cycloalkyl, Cycloalkylniedrigalkyl, Bicycloalkyl und Bicycloalkylniedrigalkyl darstellt und R₁₁ nichtsubstituiert oder substituiert ist,
. -O-R₁₀, worin R₁₀ die oben angegebenen Bedeutungen besitzt,
. Thioxo,
. -S-R₁₂, worin R₁₂ eine Niedrigalkylgruppe darstellt und nichtsubstituiert oder substituiert ist,
. Aryl,
. substituiertes Aryl und 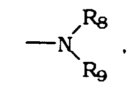 in der R₈ und R₉ unabhängig voneinander eine Gruppe ausgewählt
. Wasserstoff,
. Niedrigalkyl,
. Cycloalkyl,
. Cycloalkylniedrigalkyl,
. einen Heterocyclus R₁₃ ausgewählt aus Pyrrolidin, Piperidin, Piperazin, Morpholin und Thiomorpholin;
. oder gemeinsam mit dem sie tragenden Stickstoffatom einen Heterocyclus R₁₃ der oben definierten Art bilden, darstellen,
bedeutet,
- R₄ eine Gruppe ausgewählt aus:
. Wasserstoff,
. R₁₄, worin R₁₄ die oben angegebenen Bedeutungen von R₃ aufweist, worin R₁₄ substituiert oder nichtsubstituiert ist,
. Cycloalkyl,
. Aryl,
. substituiertes Aryl,
. Niedrigacyl,
. Niedrigalkoxycarbonyl,
. am Arylkern nichtsubstituiertes oder substituiertes Arylcarbonyl.
. am Arylkern nichtsubstituiertes oder substituiertes Aryloxycarbonyl darstellt, oder
R₄ zwischen den Atomen 2 und 3 des in der Formel (I) vorhandenen Thiadiazinrings eine Doppelbindung bildet;
- A mit den sie tragenden beiden Kohlenstoffatomen einen Pyridinkern bildet ausgewählt aus den Gruppen A₁, A₂, A₃ und A₄: 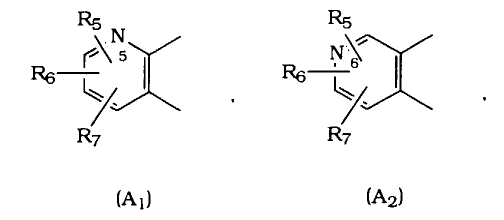 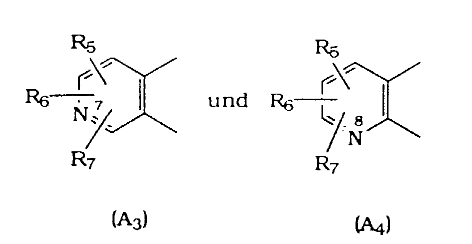 worin R₅, R₆ und R₇ unabhängig voneinander Gruppen ausgewählt aus: Wasserstoff, Halogen, Niedrigalkyl, Hydroxy. Thiol, Niedrigalkoxy, Niedrigalkylthio, Trifluormethyl, Carboxyl. Niedrigacyl, Aryl, Arylniedrigalkyl. Amino, Niedrigalkylamino und Niedrigdialkylamino bedeuten,
mit der Maßgabe, daß, wenn A eine nichtsubstituierte Gruppe der Formel A₁, R₂ ein Wasserstoffatom, eine Aminogruppe oder eine Methylgruppe und R₅, R₆ und R₇ gleichzeitig Wasserstoffatome darstellen, R₁ nicht ein Wasserstoffatom oder eine Methylgruppe bedeutet,
mit der weiteren Maßgabe, daß die Verbindungen der Formel (I) 4H-Pyrido[4,3-e]-1,2,4-thiadiazin-1,1-dioxid nicht umfassen, und wobei es sich versteht, daß, wenn nichts anderes angegeben ist,
. "Niedrigalkyl". "Niedrigalkoxy", "Niedrigacyl" und "Niedrigalkylthio" für geradkettige oder verzweigte Gruppen mit 1 bis 6 Kohlenstoffatomen stehen,
. "Niedrigalkenyl" für eine geradkettige oder verzweigte Gruppe mit 2 bis 6 Kohlenstoffatomen und 1 bis 2 Doppelbindungen steht,
. "Niedrigalkinyl" für eine geradkettige oder verzweigte Gruppe mit 2 bis 6 Kohlenstoffatomen und 1 bis 2 Dreifachbindungen steht,
. "Cycloalkyl" für eine cyclische Gruppe mit 3 bis 8 Kohlenstoffatomen steht, . "Bicycloalkyl" für eine bicyclische Gruppe mit 6 bis 12 Kohlenstoffatomen steht,
. "Aryl" für Phenyl oder Naphthyl steht,
. "substituiert" in bezug auf die Gruppen R₃, R₁₀ und R₁₄ bedeutet, daß diese Gruppen durch eine oder mehrere Gruppen ausgewählt aus Halogen. Hydroxy und Niedrigalkoxy substituiert sind,
. "substituiert" in bezug auf die Gruppen "Cycloalkyl", "Cycloalkylalkyl" und R₁₁ bedeutet, daß die in dieser Weise bezeichnete Gruppe durch einen oder mehrere Rest oder Gruppen ausgewählt aus Oxo, Hydroxy, Niedrigalkyl und Niedrigalkoxy substituiert ist,
. "substituiert" in bezug auf die Arylgruppe bedeutet, daß die in dieser Weise bezeichnete Gruppe durch eine oder mehrere Gruppen ausgewählt aus Halogen, Hydroxy, Niedrigalkyl, Niedrigalkoxy und Trifluormethyl substituiert ist,
- deren optische Isomeren
- und deren Additionssalze mit einer pharmazeutisch annehmbaren Säure oder Base.

2. Verbindung nach Anspruch 1, nämlich 4-Isopropyl-2,3-dihydro-4H-pyrido[4,3-e][1,2,4]thiadiazin-1,1-dioxid.

3. Verbindung nach Anspruch 1, nämlich 3,4-Dimethyl-4H-pyrido[4,3-e]-[1,2,4]thiadiazin-1,1-dioxid.

4. Verbindung nach Anspruch 1, nämlich 4-Methyl-2,3-dihydro-4H-pyrido-[4,3-e][1,2,4]thiadiazin-1,1-dioxid.

5. Verbindung nach Anspruch 1, nämlich 2,3-Dimethyl-2,3-dihydro-4H-pyrido[4,3-e][1,2,4]thiadiazin-1,1-dioxid.

6. Verbindung nach Anspruch 1, nämlich 4-Methyl-2,3-dihydro-4H-pyrido-[3,2-e][1,2,4]thiadiazin-1,1-dioxid.

7. Verbindung nach Anspruch 1, nämlich 4-Ethyl-2,3-dihydro-4H-pyrido-[3,2-e][1,2,4]thiadiazin-1,1-dioxid.

8. Verfahren zur Herstellung der Verbindungen der Formel (I) nach Anspruch 1, dadurch gekennzeichnet, daß man eine Verbindung der Formel (II): 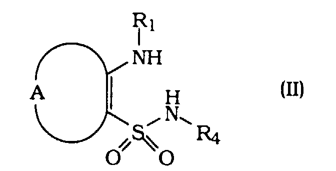 in der R₁, R₄ und A die in Anspruch 1 angegebenen Bedeutungen besitzen, a) - entweder mit einer Verbindung der Formel (III) 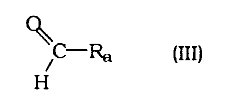 in der Rₐ eine Gruppe R₁₀, R₁₁, Aryl oder substituiertes Aryl bedeutet, wie sie in Anspruch 1 definiert sind,
oder in der Wärme mit einer Verbindung der Formel (IV): 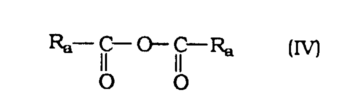 umsetzt zur Bildung der Verbindungen der Formel (I/a): 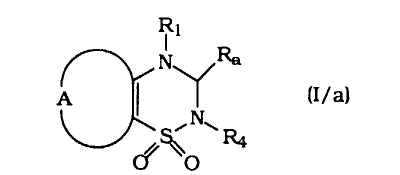 in der R₁, R₄, Rₐ und A die oben angegebenen Bedeutungen besitzen,
einem Sonderfall der Verbindungen der Formel (I), in der R₂ eine Gruppe Rₐ darstellt,
- oder mit Paraformaldehyd, Orthoameisensäureethylester oder einem gemischten Ameisensäure/Essigsäure-Anhydrid umsetzt zur Bildung der Verbindung der Formel (I/b): 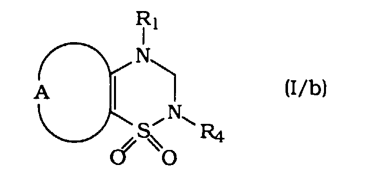 in der R₁, R₄ und A die oben angegebenen Bedeutungen besitzen,
einem Sonderfall der Verbindungen der Formel (I), in der R₂ ein Wasserstoffatom darstellt,
oder
b) - mit Harnstoff umsetzt zur Bildung der Verbindungen der Formel (V): 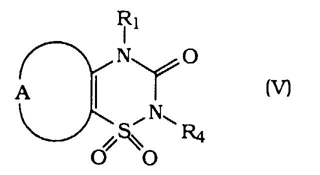 in der R₁, R₄ und A die oben angegebenen Bedeutungen besitzen, welche anschließend:
* in die Salze überführt und dann mit einer Verbindung der Formel (VI):
R₁₀ ― Hal (VI)
in der R₁₀ die in Anspruch 1 angegebenen Bedeutungen besitzt und Hal ein Halogenatom darstellt, umgesetzt werden
zur Bildung einer Verbindung der Formel (I/c): 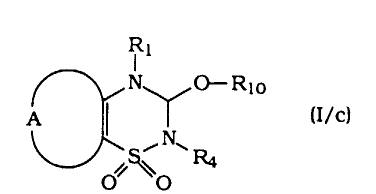 in der R₁, R₄, R₁₀ und A die oben angegebenen Bedeutungen besitzen, einem Sonderfall der Verbindungen der Formel (I), in der R₂ eine Gruppe -O-R₁₀ darstellt.
* oder mit einem Thionierungsmittel behandelt werden zur Bildung der Verbindung der Formel (I/d): 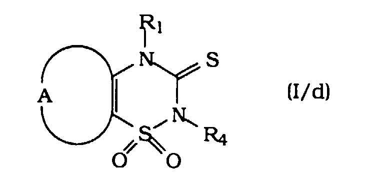 in der R₁, R₄ und A die oben angegebenen Bedeutungen besitzen, einem Sonderfall der Verbindungen der Formel (I), in der R₂ eine Thioxogruppe darstellt. welche Verbindung der Formel (I/d) mit einer Verbindung der Formel (VI):
Hal'― R₁₂ (IV)
in der R₁₂ die in Anspruch 1 angegebenen Bedeutungen besitzt und Hal' ein Halogenatom darstellt, umgesetzt wird zur Bildung der Verbindung der Formel (I/e): 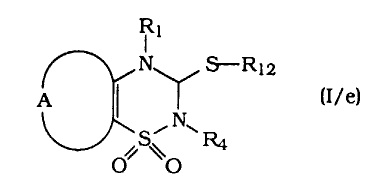 in der R₁, R₄, R₁₂ und A die oben angegebenen Bedeutungen besitzen, einem Sonderfall der Verbindungen der Formel (I), in der R₂ eine Alkylthiogruppe darstellt, welche Verbindungen der Formel (I/e) anschließend mit einem Amin der Formel (VII): 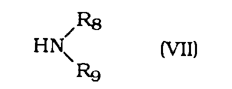 in der R₈ und R₉ die in Anspruch 1 angegebenen Bedeutungen besitzen, umgesetzt werden kann zur Bildung der Verbindungen der Formel (I/f): 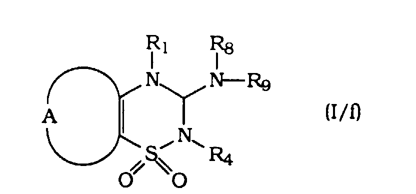 in der R₁, R₄, R₈, R₉ und A die oben angegebenen Bedeutungen besitzen,
einem Sonderfall der Verbindungen der Formel (I), in der R₂ eine Gruppe -NR₈R₉ darstellt.
welche Verbindungen der Formeln (I/a) bis (I/f) die Gesamtheit der Verbindungen der Formel (I) bilden,
wobei es sich versteht, daß die Verbindungen der Formeln (I/a), (I/b), (I/c), (I/e) und (I/f) gegebenenfalls selektiv reduziert werden können zur Bildung der entsprechenden Verbindungen der Formel (I), bei denen die Bindung zwischen den Atomen 2 und 3 oder zwischen den Atomen 3 und 4 des Thiadiazinrings gesättigt ist,
welche Verbindungen der Formel (I):
- mit Hilfe eines oder mehrerer Reinigungsverfahren ausgewählt aus Kristallisation, Säulenchromatographie über Siliciumdioxid, Extraktion, Filtration und Überführung über Aktivkohle oder ein Harz gereinigt werden können,
- in ihre eventuellen optischen Isomeren in reiner Form oder in Form einer Mischung aufgetrennt werden können
- und mit einer pharmazeutisch annehmbaren Säure oder Base in ihre Salze überführt werden können.

9. Verfahren zur Herstellung der Verbindungen der Formel (I/a), einem Sonderfall der Verbindungen der Formel (I) nach Anspruch 1: 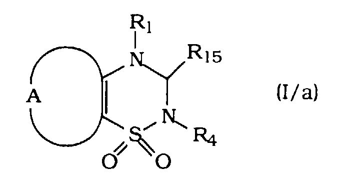 in der R₁, R₄ und A die in Anspruch 1 angegebenen Bedeutungen besitzen und R₁₅ eine Gruppe R₁₀ oder R₁₁, wie sie in Anspruch 1 definiert sind, darstellt,
dadurch gekennzeichnet, daß man
eine Verbindung der Formel (II/a): 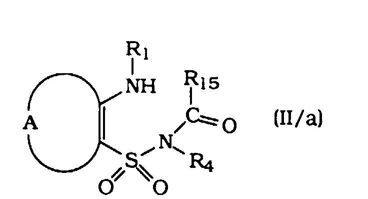 in der R₁, R₄, R₁₅ und A die oben angegebenen Bedeutungen besitzen, mit einem Cyclisierungsmittel behandelt
zur Bildung der entsprechenden Verbindung der Formel (I/a),
welche in dieser Weise erhaltenen Verbindungen der Formel (I/a)
- mit Hilfe eines oder mehrerer Reinigungsverfahren ausgewählt aus Kristallisation, Säulenchromatographie über Siliciumdioxid, Extraktion, Filtration und Überführung über Aktivkohle oder ein Harz gereinigt werden können,
- in ihre eventuellen optischen Isomeren in reiner Form oder in Form einer Mischung aufgetrennt werden können
- und mit einer pharmazeutisch annehmbaren Säure oder Base in ihre Salze überführt werden können.

10. Verfahren zur Herstellung der Verbindungen der Formel (I/g), einem Sonderfall der Verbindungen der Formel (I) nach Anspruch 1: 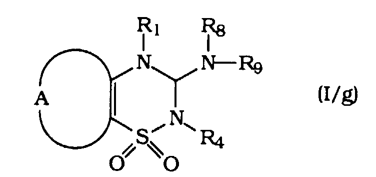 in der R₄, R₈, R₉ und A die in Anspruch 1 angegebenen Bedeutungen besitzen, einem Sonderfall der Verbindungen der Formel (I), in der R₂ eine Gruppe -NR₈R₉ darstellt und R₁ ein Wasserstoffatom bedeutet,
dadurch gekennzeichnet, daß man:
- entweder eine Verbindung der Formel (II/b): 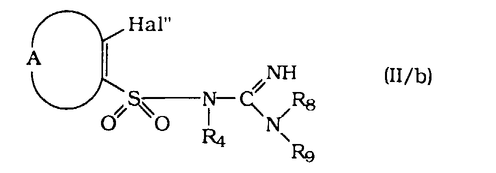 in der R₄, R₈, R₉ und A die oben angegebenen Bedeutungen besitzen und Hal" ein Halogenatom darstellt, im basischem Medium in der Wärme umsetzt,
- oder eine Verbindung der Formel (II): 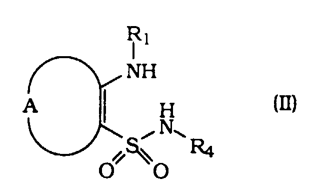 in der R₁, R₄ und A die oben angegebenen Bedeutungen besitzen, mit einer Verbindung der Formel (VIII): 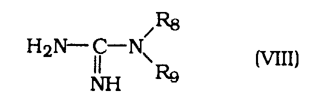 in der R₈ und R₉ die oben angegebenen Bedeutungen besitzen, umsetzt zur Bildung der entsprechenden Verbindung der Formel (I/g),
- mit Hilfe eines oder mehrerer Reinigungsverfahren ausgewählt aus Kristallisation, Säulenchromatographie über Siliciumdioxid, Extraktion, Filtration und Überführung über Aktivkohle oder ein Harz gereinigt werden können,
- in ihre eventuellen optischen Isomeren in reiner Form oder in Form einer Mischung aufgetrennt werden können
- und mit einer pharmazeutisch annehmbaren Säure oder Base in ihre Salze überführt werden können.

11. Verfahren zur Herstellung der Verbindungen der Formel (I/h), einem Sonderfall der Verbindungen der Formel (I) nach Anspruch 1: 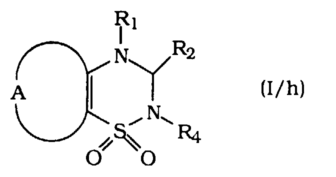 in der R₁, R₄ und A die in Anspruch 1 angegebenen Bedeutungen besitzen, wobei es sich versteht, daß mindestens einer der Substituenten R₁ oder R₄ von einem Wasserstoffatom verschieden ist,
dadurch gekennzeichnet, daß man
eine Verbindung der Formel (I/i): 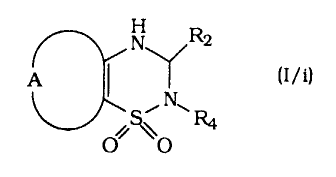 in der R_{2,} R₄ und A die oben angegebenen Bedeutungen besitzen, mit einer Verbindung der Formel (XI):
R₁' ― X (XI)
in der R₁' die in Anspruch 1 angegebenen Bedeutungen von R₁ besitzt mit Ausnahme des Wasserstoffatoms, und X eine austretende Gruppe darstellt, umsetzt und/oder wenn R₄ ein Wasserstoffatom darstellt, mit einer Verbindung der Formel (XII):
R₄' ― X' (XII)
in der R₄' die in Anspruch 1 angegebenen Bedeutungen von R₄ besitzt mit Ausnahme des Wasserstoffatoms, und X' eine austretende Gruppe darstellt, umsetzt,
- mit Hilfe eines oder mehrerer Reinigungsverfahren ausgewählt aus Kristallisation, Säulenchromatographie über Siliciumdioxid, Extraktion, Filtration und Überführung über Aktivkohle oder ein Harz gereinigt werden können,
- in ihre eventuellen optischen Isomeren in reiner Form oder in Form einer Mischung aufgetrennt werden können
- und mit einer pharmazeutisch annehmbaren Säure oder Base in ihre Salze überführt werden können.

12. Pharmazeutische Zubereitung enthaltend eine Verbindung nach Anspruch 1 oder eines ihrer Salze mit einer pharmazeutisch annehmbaren Säure oder Base in Kombination mit einem oder mehreren pharmazeutisch annehmbaren Trägermaterialien oder Bindemitteln.

13. Pharmazeutische Zubereitung nach Anspruch 12 enthaltend eine Verbindung nach Anspruch 1 oder eines ihrer Salze mit einer pharmazeutisch annehmbaren Säure oder Base zur Behandlung und zur Vorbeugung von pathologischen Zuständen, die mit einer Fehlfunktion der glutamatergischen Neurotransmission verknüpft sind.

14. Pharmazeutische Zubereitung nach Anspruch 13 zur Behandlung und zur Vorbeugung von mit dem Alter verbundenen Gedächtnis/Erkenntnis-Störungen, von Angst- oder Depressionssyndromen, von progressiven neurodegenerativen Erkrankungen, der Alzheimerschen Krankheit, der Pickschen Krankheit, der Huntington Chorea, der Schizophrenie, von den Folgen von akuten neurodegenerativen Erkrankungen, den Folgen der Ischämie und den Folgen der Epilepsie.

## Claims

1. Compounds of formula (I): 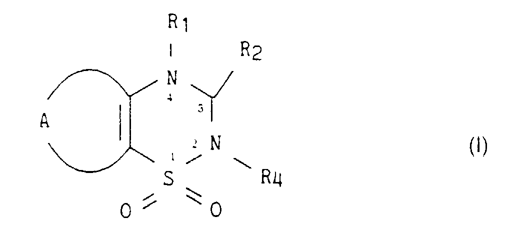 in which:
- R₁ represents a group selected from:
. hydrogen,
. R₃, with R₃ representing a radical selected from lower alkyl, lower alkenyl and lower alkynyl, R₃ being unsubstituted or substituted,
. cycloalkyl,
. substituted cycloalkyl,
. cycloalkyl-lower alkyl,
. substituted cycloalkyl-lower alkyl,
. lower acyl,
. lower alkoxycarbonyl,
. arylcarbonyl that is unsubstituted or substituted on the aryl ring, and
. aryloxycarbonyl that is unsubstituted or substituted on the aryl ring,
or R₁ forms a double bond between atoms 3 and 4 of the thiadiazine ring present in formula (I);
- R₂ represents a group selected from:
. hydrogen,
. R₁₀, with R₁₀ having the same definition as R₃ above, R₁₀ being unsubstituted or substituted,
. R₁₁, with R₁₁ representing a group selected from cycloalkyl, cycloalkyl-lower alkyl, bicycloalkyl and bicycloalkyl-lower alkyl, R₁₁ being unsubstituted or substituted,
. -O-R₁₀, wherein R₁₀ is as defined above,
. thioxo,
. -S-R₁₂, wherein R₁₂ represents a lower alkyl radical, R₁₂ being unsubstituted or substituted,
. aryl,
. substituted aryl, and 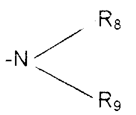 wherein R₈ and R₉ each independently of the other represents a radical selected from:
. hydrogen,
. lower alkyl,
. cycloalkyl,
. cycloalkyl-lower alkyl, and
. a heterocycle R₁₃ selected from pyrrolidine, piperidine, piperazine, morpholine and thiomorpholine,
. or R₈ and R₉ together with the nitrogen atom carrying them form a heterocycle R₁₃ as defined above;
- R₄ represents a group selected from:
. hydrogen,
. R₁₄, with R₁₄ having the same definition as R₃ above, R₁₄ being unsubstituted or substituted,
. cycloalkyl,
. aryl,
. substituted aryl,
. lower acyl,
. lower alkoxycarbonyl,
. arylcarbonyl that is unsubstituted or substituted on the aryl ring, and
. aryloxycarbonyl that is unsubstituted or substituted on the aryl ring,
or R₄ forms a double bond between atoms 2 and 3 of the thiadiazine ring present in formula (I);
- A with the two carbon atoms carrying it forms a pyridine ring selected from the groups A₁, A₂, A₃ and A₄: 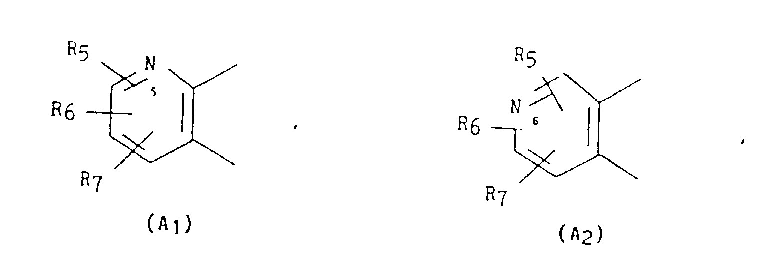 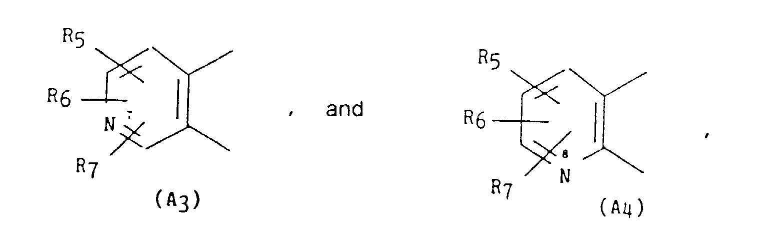 in which R₅, R₆ and R₇ each independently of the others represents a radical selected from: hydrogen, halogen, lower alkyl, hydroxy, thiol, lower alkoxy, lower alkylthio, trifluoromethyl, carboxy, lower acyl, aryl, aryl-lower alkyl, amino, lower alkylamino and di-lower alkylamino,
with the proviso that when A represents an unsubstituted group of the formula A₁, R₂ represents a hydrogen atom, an amino radical or a methyl radical, and R₅, R₆ and R₇ simultaneously represent hydrogen atoms, then R₁ may not be a hydrogen atom or a methyl radical,
with the proviso that the compound of formula (I) may not be 4H-pyrido[4,3-e]-1,2,4-thiadiazine 1,1-dioxide,
wherein, unless indicated to the contrary:
- "lower alkyl", "lower alkoxy", "lower acyl" and "lower alkylthio" denote linear or branched groups having from 1 to 6 carbon atoms,
- "lower alkenyl" denotes a linear or branched group having from 2 to 6 carbon atoms containing 1 or 2 double bonds,
- "lower alkynyl" denotes a linear or branched group having from 2 to 6 carbon atoms containing 1 or 2 triple bonds,
- "cycloalkyl" denotes a cyclic group having from 3 to 8 carbon atoms,
- "bicycloalkyl" denotes a bicyclic group having from 6 to 12 carbon atoms,
- "aryl" denotes phenyl or naphthyl,
- "substituted" associated with the groups R₃, R₁₀ and R₁₄ means that those groups so qualified are substituted by one or more radicals selected from halogen, hydroxy and lower alkoxy,
- "substituted" associated with the groups "cycloalkyl", "cycloalkylalkyl" and R₁₁ means that those groups so qualified are substituted by one or more radicals or groups selected from oxo, hydroxy, lower alkyl and lower alkoxy,
- "substituted" associated with the aryl group means that that group so qualified is substituted by one or more radicals selected from halogen, hydroxy, lower alkyl, lower alkoxy and trifluoromethyl,
- their optical isomers,
- and addition salts thereof with a pharmaceutically acceptable acid or base.

2. Compound according to claim 1 which is 4-isopropyl-2,3-dihydro-4H-pyrido-[4,3-e][1,2,4]thiadiazine 1,1-dioxide.

3. Compound according to claim 1 which is 3,4-dimethyl-4H-pyrido[4,3-e][1,2,4]-thiadiazine 1,1-dioxide.

4. Compound according to claim 1 which is 4-methyl-2,3-dihydro-4H-pyrido[4,3-e]-[1,2,4]thiadiazine 1,1-dioxide.

5. Compound according to claim 1 which is 2,3-dimethyl-2,3-dihydro-4H-pyrido-[4,3-e][1,2,4]thiadiazine 1,1-dioxide.

6. Compound according to claim 1 which is 4-methyl-2,3-dihydro-4H-pyrido[3,2-e]-[1,2,4]thiadiazine 1,1-dioxide.

7. Compound according to claim 1 which is 4-ethyl-2,3-dihydro-4H-pyrido[3,2-e]-[1,2,4]thiadiazine 1,1-dioxide.

8. Process for the preparation of compounds of formula (I) according to claim 1, characterised in that a compound of formula (II): 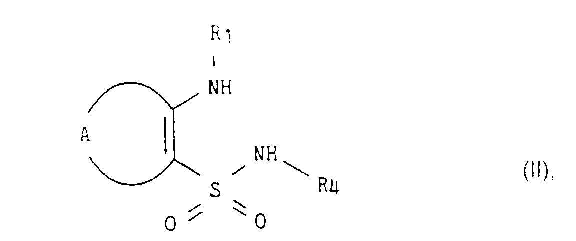 in which R₁, R₄ and A are as defined in claim 1,
is reacted
a) either with a compound of formula (III): 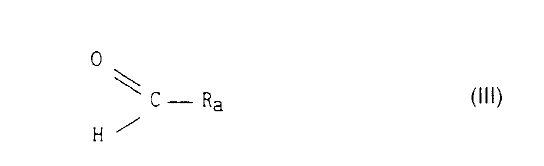 in which Rₐ represents an R₁₀, R₁₁, aryl or substituted aryl group, as defined in claim 1,
or, while heating, with a compound of formula (IV): 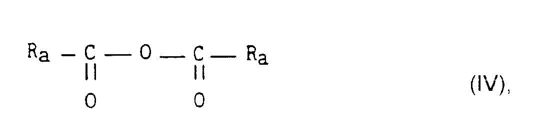 to give a compound of formula (I/a): 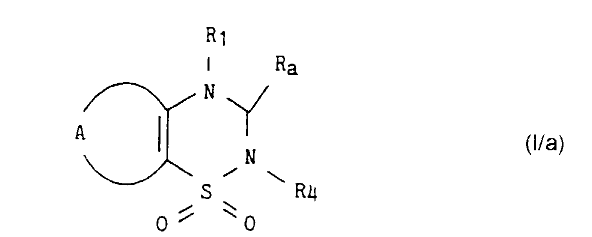 in which R₁, R₄, Rₐ and A are as defined above, which is a particular case of the compounds of formula (I) in which R₂ represents a group Rₐ,
or with paraformaldehyde, ethyl orthoformate or a formic acid/acetic acid mixed anhydride, to give a compound of formula (I/b): 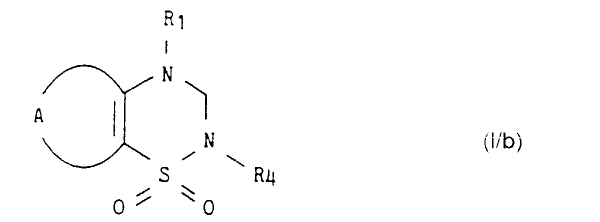 in which R₁, R₄ and A are as defined above, which is a particular case of the compounds of formula (I) in which R₂ represents a hydrogen atom,
or alternatively
b) with urea to yield a compound of formula (V): 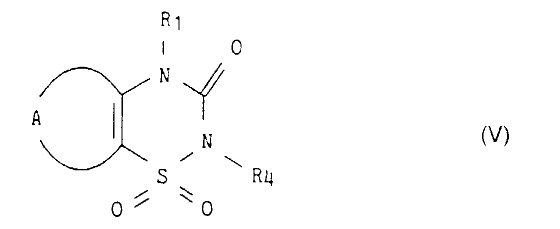 in which R₁, R₄ and A are as described above,
which is then:
* converted into a salt and then reacted with a compound of formula (VI):
R₁₀-Hal (VI)
in which R₁₀ is as defined in claim 1 and Hal represents a halogen atom, to give a compound of formula (I/c): 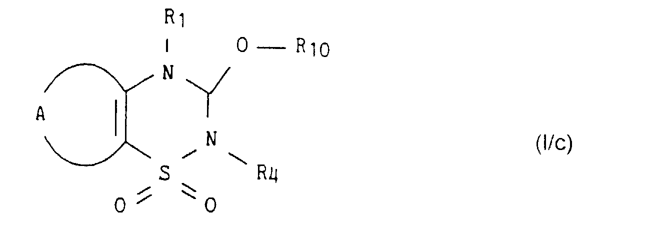 in which R₁, R₄, R₁₀ and A are as defined above, which is a particular case of the compounds of formula (I) in which R₂ represents a group -O-R₁₀,
* or subjected to a thionating agent to give a compound of formula (I/d): 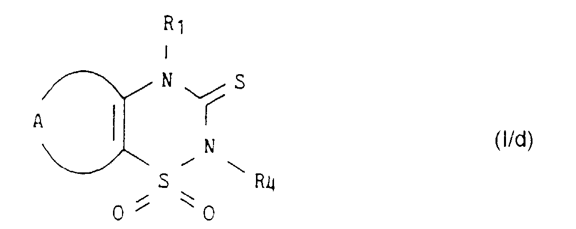 in which R₁, R₄ and A are as defined above, which is a particular case of the compounds of formula (I) in which R₂ represents a thioxo group,
which compound of formula (I/d) is reacted with a compound of formula (VI):
Hal'-R₁₂ (VI)
in which R₁₂ is as defined in claim 1 and Hal' represents a halogen atom, to give a compound of formula (I/e): 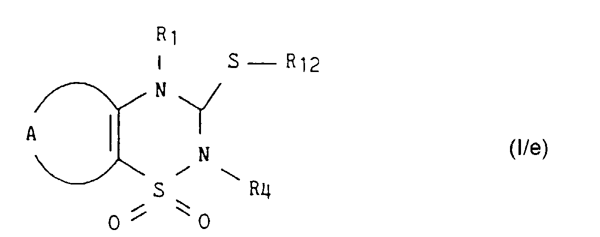 in which R₁, R₄, R₁₂ and A are as defined above, which is a particular case of the compounds of formula (I) in which R₂ represents an alkylthio group,
which compound of formula (I/e) may then be subjected to an amine of formula (VII): 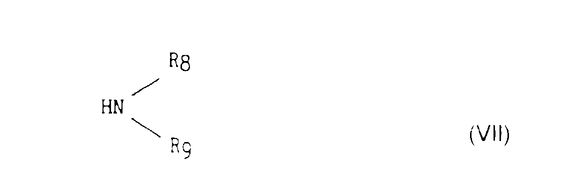 in which R₈ and R₉ are as defined in claim 1,
to give a compound of formula (I/F): 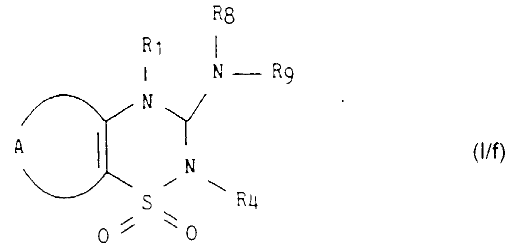 in which R₁, R₄, R₈, R₉ and A are as defined above, which is a particular case of the compounds of formula (I) in which R₂ represents a group -NR₈R₉,
the compounds of formulae (I/a) to (I/f) forming the totality of the compounds of formula (I),
it being understood that the compounds of formulae (I/a), (I/b), (I/c), (I/e) and (I/f) may optionally be reduced selectively in order to obtain the corresponding compounds of formula (I) wherein the bond between atoms 2 and 3, or between atoms 3 and 4, of the thiadiazine ring is saturated,
which compounds of formula (I) may be:
- purified by one or more purification methods selected from crystallisation, chromatography on a silica column, extraction, filtration, and passage over charcoal or resin,
- separated, in pure form or in the form of a mixture, into their possible optical isomers,
- and converted into a salt by means of a pharmaceutically acceptable acid or base.

9. Process for the preparation of compounds of formula (I/a), a particular case of the compounds of formula (I) according to claim 1: 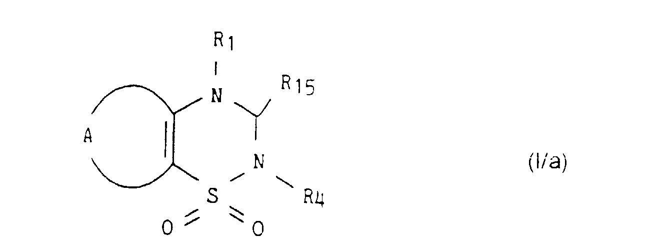 in which R₁, R₄ and A are as defined in claim 1 and R₁₅ represents a group R₁₀ or R₁₁ as defined in claim 1,
characterised in that:
a compound of formula (II/a): 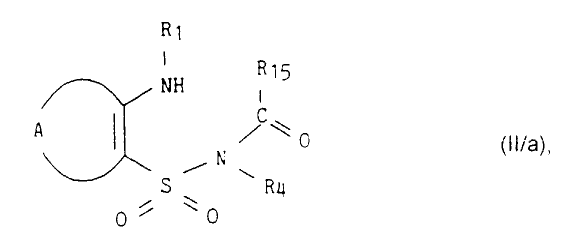 in which R₁, R₄, R₁₅ and A are as defined above,
is subjected to a cyclising agent in order to obtain the corresponding compound of formula (I/a),
which compounds of formula (I/a) so obtained may be:
- purified by one or more purification methods selected from crystallisation, chromatography on a silica column, extraction, filtration, and passage over charcoal or resin,
- separated, in pure form or in the form of a mixture, into their possible optical isomers,
- and converted into a salt by means of a pharmaceutically acceptable acid or base.

10. Process for the preparation of compounds of formula (I/g), a particular case of the compounds of formula (I) according to claim 1: 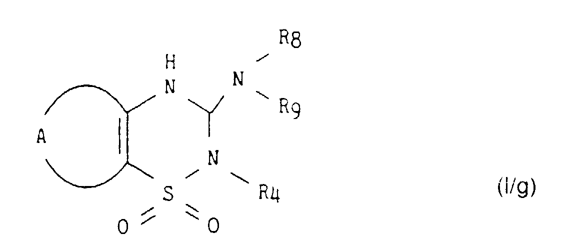 in which R₄, R₈, R₉ and A are as defined in claim 1, which are a particular case of the compounds of formula (I) in which R₂ represents a group -NR₈R₉ and R₁ represents a hydrogen atom,
characterised in that:
- either a compound of formula (II/b): 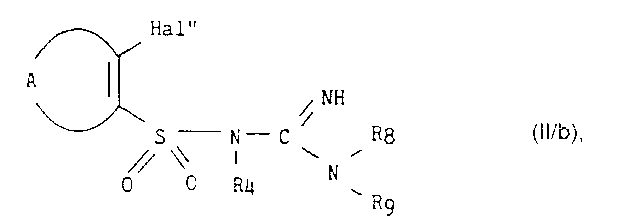 in which R₄, R₈, R₉ and A are as defined above and Hal" represents a halogen atom, is reacted, while heating, in a basic medium,
- or a compound of formula (II): 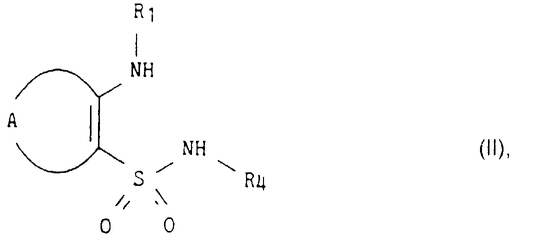 in which R₁, R₄ and A are as defined above,
is reacted with a compound of formula (VIII): 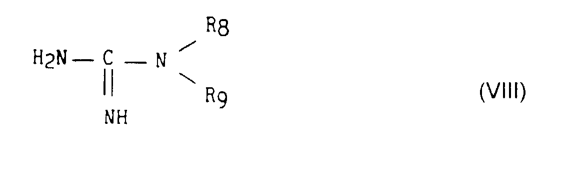 in which R₈ and R₉ are as defined above,
in order to obtain the corresponding compound of formula (I/g),
which compounds of formula (I/g) may be:
- purified by one or more purification methods selected from crystallisation, chromatography on a silica column, extraction, filtration, and passage over charcoal or resin,
- separated, in pure form or in the form of a mixture, into their possible optical isomers,
- and converted into a salt by means of a pharmaceutically acceptable acid or base.

11. Process for the preparation of compounds of formula (I/h), a particular case of the compounds of formula (I) according to claim 1: 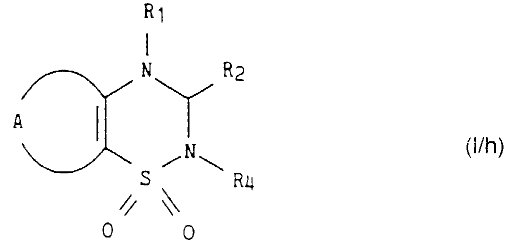 in which R₁, R₄ and A are as defined in claim 1, with the proviso that at least one of the substituents R₁ and R₄ is other than a hydrogen atom,
characterised in that:
a compound of formula (I/i): 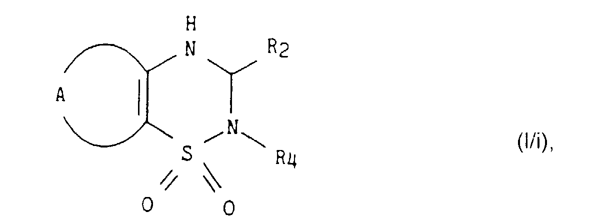 in which R₂, R₄ and A are as defined above,
is reacted with a compound of formula (XI):
R₁'-X (XI)
in which R₁' has the same meanings as R₁ as defined in claim 1, with the exception of hydrogen, and X represents a leaving group,
and/or, when R₄ represents a hydrogen atom, with a compound of formula (XII):
R₄'-X' (XII)
in which R₄' has the same meanings as R₄ as defined in claim 1, with the exception of hydrogen, and X' represents a leaving group,
it being possible for the compounds of formula (I/h) so obtained to be:
- purified by one or more purification methods selected from crystallisation, chromatography on a silica column, extraction, filtration, and passage over charcoal or resin,
- separated, in pure form or in the form of a mixture, into their possible optical isomers,
- and converted into a salt by means of a pharmaceutically acceptable acid or base.

12. Pharmaceutical composition comprising a compound according to claim 1 or a salt thereof with a pharmaceutically acceptable acid or base, in combination with one or more pharmaceutically acceptable excipients or carriers.

13. Pharmaceutical composition according to claim 12 comprising a compound according to claim 1 or a salt thereof with a pharmaceutically acceptable acid or base, for use in the treatment and prevention of pathologies associated with dysfunctioning of glutamatergic neurotransmission.

14. Pharmaceutical composition according to claim 13 for use in the treatment and prevention of mnemocognitive disorders associated with age and with anxiety or depressive syndromes, of progressive neurodegenerative diseases, of Alzheimer's disease, of Pick's disease, of Huntington's chorea, of schizophrenia, of the after-effects of acute neurodegenerative diseases, of the after-effects of ischaemia, and of the after-effects of epilepsy.
